# EUROPEAN PATENT APPLICATION

(11) **EP 3 395 268 A1**
(43) Date of publication of application: **31.10.2018**
(21) Application number: 18163041.9
(22) Date of filing: 21.03.2018
(51) Int. Cl.: A61B 17/128

(54) **ENDOSCOPIC SURGICAL CLIP APPLIER**

(30) Priority: 22.03.2017 US 201762474820 P; 05.01.2018 US 201815863827
(71) Applicant: Covidien LP, Mansfield, MA 02048 (US)
(72) Inventor: ZAMMATARO, Thomas, Hamden, Connecticut 06517 (US)
(74) Representative: Soames, Candida Jane

(57) **Abstract**

A reposable surgical clip applier is provided and includes a shaft assembly having an elongated shaft and a housing assembly. The housing assembly is disposed about a proximal portion of the elongated shaft and includes a housing and a hub that is operably coupled to the elongated shaft. The housing defines a counterbore. The hub is slidably disposed within the counterbore and defines a throughhole configured to receive the elongated shaft therein. A force applied to the elongated shaft in a distal direction causes the hub to distally advance within the housing. A shaft assembly for use with a reposable surgical clip applier is also provided.

## Description

### CROSS-REFERENCE TO RELATED APPLICATIONS

This application claims the benefit of and priority to U.S. Provisional Patent Application No. 62/474,820 filed March 22, 2017, the entire disclosure of which is incorporated by reference herein.

### BACKGROUND

### Technical Field

The technical field relates to surgical clip appliers. More particularly, the present disclosure relates to endoscopic surgical clip appliers having handle assemblies configured for use with various different endoscopic assemblies.

### Description of Related Art

Endoscopic surgical staplers and surgical clip appliers are known in the art and are used for a number of distinct and useful surgical procedures. In the case of a laparoscopic surgical procedure, access to the interior of an abdomen is achieved through narrow tubes or cannulas inserted through a small entrance incision in the skin. Minimally invasive procedures performed elsewhere in the body are often generally referred to as endoscopic procedures. Typically, a tube or cannula device is extended into the patient's body through the entrance incision to provide an access port. The port allows the surgeon to insert a number of different surgical instruments therethrough using a trocar and for performing surgical procedures far removed from the incision.

During a majority of these procedures, the surgeon must often terminate the flow of blood or another fluid through one or more vessels. The surgeon will often use a particular endoscopic surgical clip applier to apply a surgical clip to a blood vessel or another duct to prevent the flow of body fluids therethrough during the procedure.

Endoscopic surgical clip appliers having various sizes (e.g., diameters), that are configured to apply a variety of diverse surgical clips, are known in the art, and which are capable of applying a single or multiple surgical clips during an entry to the body cavity. Such surgical clips are typically fabricated from a biocompatible material and are usually compressed over a vessel. Once applied to the vessel, the compressed surgical clip terminates the flow of fluid therethrough.

Endoscopic surgical clip appliers that are able to apply multiple clips in endoscopic or laparoscopic procedures during a single entry into the body cavity are described in commonly-assigned U.S. Pat. Nos. 5,084,057 and 5,100,420 to Green et al., which are both incorporated by reference in their entirety. Another multiple endoscopic surgical clip applier is disclosed in commonly-assigned U.S. Pat. No. 5,607,436 by Pratt et al., the contents of which is also hereby incorporated by reference herein in its entirety. These devices are typically, though not necessarily, used during a single surgical procedure. U.S. Pat. No. 5,695,502 to Pier et al., the disclosure of which is hereby incorporated by reference herein, discloses a resterilizable endoscopic surgical clip applier. The endoscopic surgical clip applier advances and forms multiple clips during a single insertion into the body cavity. This resterilizable endoscopic surgical clip applier is configured to receive and cooperate with an interchangeable clip magazine so as to advance and form multiple clips during a single entry into a body cavity.

During endoscopic or laparoscopic procedures it may be desirable and/or necessary to use different size surgical clips or different configured surgical clips depending on the underlying tissue or vessels to be ligated. In order to reduce overall costs of an endoscopic surgical clip applier, it is desirable for a single endoscopic surgical clip applier to be loadable with and capable of firing different size surgical clips as needed.

Accordingly, a need exists for endoscopic surgical clip appliers that include handle assemblies configured for use with various different endoscopic assemblies having different clips loaded therein and/or configured for performing various different surgical tasks.

### SUMMARY

As detailed herein and shown in the drawing figures, as is traditional when referring to relative positioning on a surgical instrument, the term "proximal" refers to the end of the apparatus or component thereof which is closer to the user and the term "distal" refers to the end of the apparatus or component thereof which is further away from the user. Further, to the extent consistent, any or all of the aspects and features detailed herein may be used in conjunction with any or all of the other aspects and features detailed herein.

Provided in accordance with aspects of the present disclosure is a reposable surgical clip applier including a shaft assembly having an elongated shaft and a housing assembly disposed about a proximal portion of the elongated shaft. The housing assembly includes a housing and a hub. The housing defines a counterbore and the hub is slidably disposed within the counter bore. The hub defines a throughhole configured to receive the elongate shaft therein and is operably coupled to the elongated shaft. A force applied to the elongated shaft in a distal direction causes the hub to distally advance within the housing.

The housing assembly may further include a biasing element interposed between the housing and the hub that includes a predetermined spring constant.

In embodiments, when a force applied to the elongated shaft exceeds the predetermined spring constant the hub may be distally advanced within the housing and compress the biasing element.

The shaft assembly may further include first and second jaw members pivotably disposed on a distal portion of the elongated shaft that are configured to transition between a first, open position, to a second, approximated position.

In embodiments, when the first and second jaw members are inhibited from transition to the second, approximated position, the elongated shaft and hub may exert a force on the biasing element that exceeds the predetermined spring constant to distally advance the hub within the housing.

The reposable surgical clip applier may further include a handle assembly that includes a housing and a trigger rotatably supported on the handle assembly. The trigger is in mechanical communication with the first and second jaw members.

The hub may be configured to distally advance a predetermined distance to permit the trigger to return to a first, unactuated position, and therefore, permit the first and second jaw members to return to the first, open, position.

The biasing element may include a predetermined spring constant that is greater than a force required to form a surgical clip disposed within the first and second jaw members.

The housing may define a pair of transverse through-holes configured to fixedly receive a pair of pins.

The hub may define a pair of transverse channels configured to slidably receive the pair of pins.

A proximal portion of the elongated shaft may define a flare configured to abut a chamfer defined on a proximal portion of the hub. The flare is configured to inhibit distal translation of the elongated shaft relative to the hub.

According to another aspect of the present disclosure, a shaft assembly for use with a reposable surgical clip applier is provided. The shaft assembly includes an elongated shaft and a housing assembly that is disposed on a proximal portion of the elongated shaft. The housing assembly includes a housing and a hub that is operably coupled to the elongated shaft. The housing defines a counterbore and the hub is slidably disposed within the counterbore. The hub defines a throughhole configured to receive the elongated shaft therein. A force applied to the elongated shaft in a distal direction causes the hub to distally advance within the housing.

The housing assembly may further include a biasing element interposed between the housing and the hub that includes a predetermined spring constant.

In embodiments, when a force applied to the elongated shaft exceeds the predetermined spring constant the hub may be distally advanced within the housing and compresses the biasing element.

The shaft assembly may further include first and second jaw members pivotably disposed on a distal portion of the elongated shaft that are configured to transition between a first, open position, to a second, approximated position.

In embodiments, when the first and second jaws are inhibited from transition to the second, approximated position, the elongated shaft and hub may exert a force on the biasing element that exceeds the predetermined spring constant to distally advance the hub within the housing.

The biasing element may include a predetermined spring constant that is greater than a force required to form a surgical clip disposed within the first and second jaw members.

The housing may define a pair of transverse through-holes configured to fixedly retain a pair of pins.

The hub may define a pair of transverse channels configured to slidably receive the pair of pins.

A proximal portion of the elongated shaft may define a flare configured to abut a chamfer defined on a proximal portion of the hub. The flare is configured to inhibit distal translation of the elongated shaft relative to the hub.

### BRIEF DESCRIPTION OF THE DRAWINGS

Aspects and features of the presently-disclosed endoscopic surgical clip applier are described in detail with reference to the drawing figures wherein like reference numerals identify similar or identical structural elements and:
FIG. 1 is a perspective view of the proximal portion of an endoscopic surgical clip applier provided in accordance with the present disclosure including a handle assembly having an endoscopic assembly engaged therewith;
FIG. 2 is perspective view of the endoscopic surgical clip applier of FIG. 1 with the endoscopic assembly removed from the handle assembly;
FIG. 3 is an enlarged, perspective view of the area of detail indicated as "3" in FIG. 2;
FIG. 4 is a transverse, cross-sectional view taken across section line 4-4 in FIG. 3;
FIG. 5 is a transverse, cross-sectional view taken across section line 5-5 in FIG. 3;
FIG. 6 is a transverse, cross-sectional view taken across section line 6-6 in FIG. 1;
FIG. 7 is a longitudinal, cross-sectional view taken across section line 7-7 in FIG. 6;
FIG. 8 is a longitudinal, cross-sectional view of handle assembly of FIG. 1;
FIG. 9 is an exploded view of the handle assembly of FIG. 1;
FIG. 10 is a perspective view of the handle assembly of FIG. 1 with a portion of the housing removed to illustrate the internal components therein;
FIG. 11 is a perspective view of the internal assemblies of the handle assembly of FIG. 1;
FIG. 12 is an enlarged, longitudinal, cross-sectional view of the area of detail indicated as "12" in FIG. 8;
FIG. 13 is an enlarged, perspective view of the area of detail indicated as "13" in FIG. 10;
FIG. 14 is an enlarged, perspective view of the area of detail indicated as "14" in FIG. 11;
FIG. 15 is a perspective view of another endoscopic assembly configured for use with the handle assembly of FIG. 1;
FIG. 16 is an enlarged, perspective view of the distal portion of the endoscopic assembly of FIG. 15;
FIG. 17 is an enlarged, perspective view of the proximal portion of the endoscopic assembly of FIG. 15;
FIG. 18 is an enlarged, perspective, of the proximal portion of the endoscopic assembly of FIG. 15 with a portion of the outer housing shown in phantom to illustrate the internal components therein;
FIG. 19 is a longitudinal, cross-sectional view of the endoscopic assembly of FIG. 15;
FIG. 20 is an enlarged, longitudinal, cross-sectional view of the proximal portion of the endoscopic assembly of FIG. 15;
FIG. 21 is an enlarged, longitudinal, cross-sectional view illustrating the operable engagement between the handle assembly of FIG. 1 and the endoscopic assembly of FIG. 15;
FIG. 22 is a perspective view of another endoscopic assembly configured for use with the handle assembly of FIG. 1;
FIG. 23 is an enlarged, perspective view of the distal portion of the endoscopic assembly of FIG. 22;
FIG. 24 is an enlarged, perspective view of the proximal portion of the endoscopic assembly of FIG. 22;
FIG. 25 is an enlarged, perspective, of the proximal portion of the endoscopic assembly of FIG. 22 with a portion of the outer housing shown in phantom to illustrate the internal components therein;
FIG. 26 is a longitudinal, cross-sectional view of the endoscopic assembly of FIG. 22;
FIG. 27 is a longitudinal, cross-sectional view of the proximal portion of the endoscopic assembly of FIG. 22;
FIG. 28 is an enlarged, longitudinal, cross-sectional view illustrating the operable engagement between the handle assembly of FIG. 1 and the endoscopic assembly of FIG. 22;
FIG. 29 is a top, cross-sectional view illustrating an alternate embodiment of an endoscopic assembly provided in accordance with the present disclosure;
FIG. 30 is a perspective view of yet another embodiment of an endoscopic assembly provided in accordance with the present disclosure;
FIG. 31 is a cross-sectional view of the endoscopic assembly of FIG. 30, taken along section line 31-31;
FIG. 32 is an enlarged view of the area of detail indicated in FIG. 31;
FIG. 32A is a side, cross-sectional view, of a housing of the endoscopic assembly of FIG. 30;
FIG. 32B is a side, cross-sectional view, of a hub of the endoscopic assembly of FIG. 30;
FIG. 33 is an rear, perspective view, of the endoscopic assembly of FIG. 30 illustrated with the housing removed; and
FIG. 34 is a schematic illustration of a robotic surgical system configured for use in accordance with the present disclosure.

### DETAILED DESCRIPTION OF EMBODIMENTS

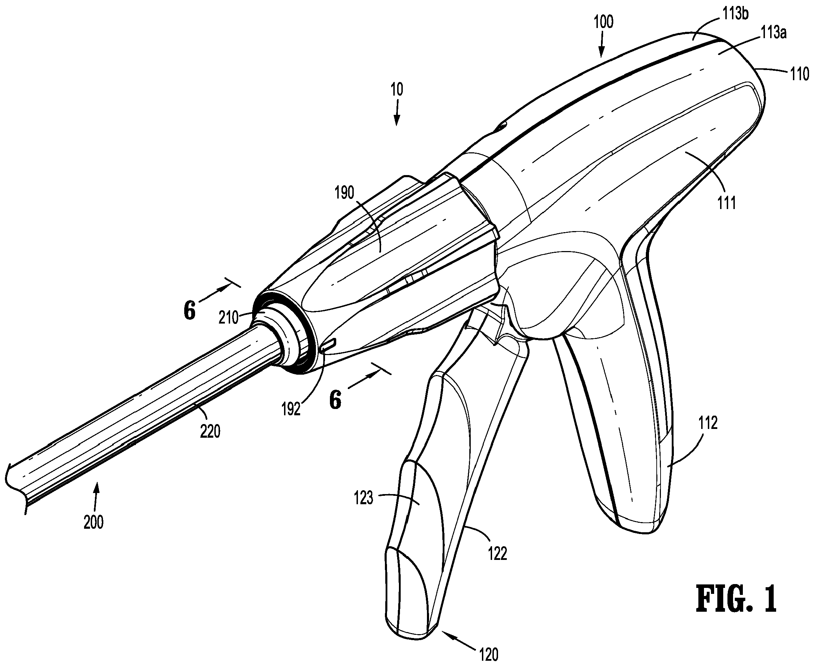

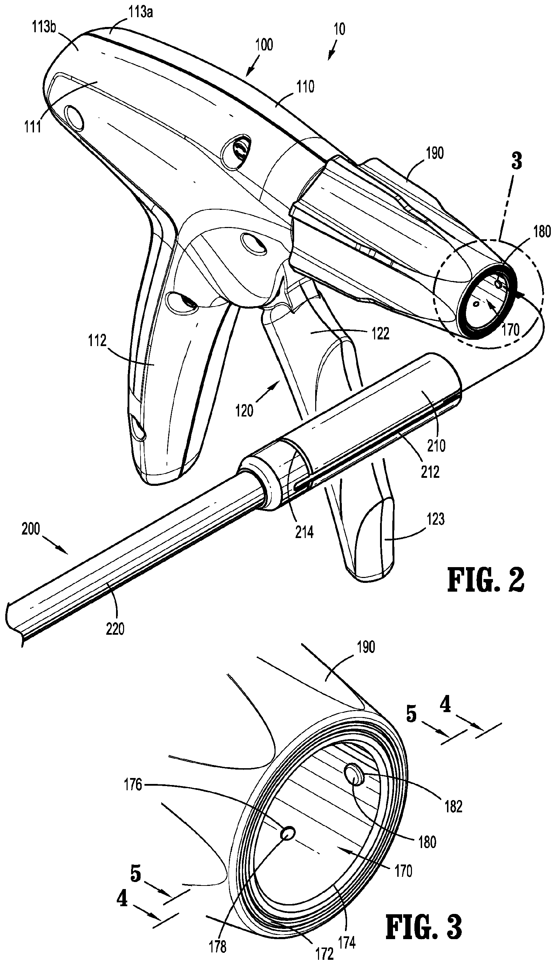

Turning to FIGS. 1 and 2, an endoscopic surgical clip applier provided in accordance with the present disclosure is identified by reference numeral 10. Surgical clip applier 10 generally includes a handle assembly 100 and a plurality of endoscopic assemblies 200 selectively connectable to and extendable distally from handle assembly 100. Handle assembly 100 is advantageously configured to operate each of the plurality of endoscopic assemblies 200, upon connection thereto, and may be configured as a sterilizable, reusable component such that handle assembly 100 may be repeatedly used with different and/or additional endoscopic assemblies 200 during the course of one or more surgical procedures. The endoscopic assemblies 200 may be configured as single-use disposable components, limited-use disposable components, or reusable components, depending upon a particular purpose and/or the configuration of the particular endoscopic assembly 200. In either configuration, the need for multiple handle assemblies 100 is obviated and, instead, the surgeon need only select an appropriate endoscopic assembly 200 and connect that endoscopic assembly 200 to handle assembly 100 in preparation for use.

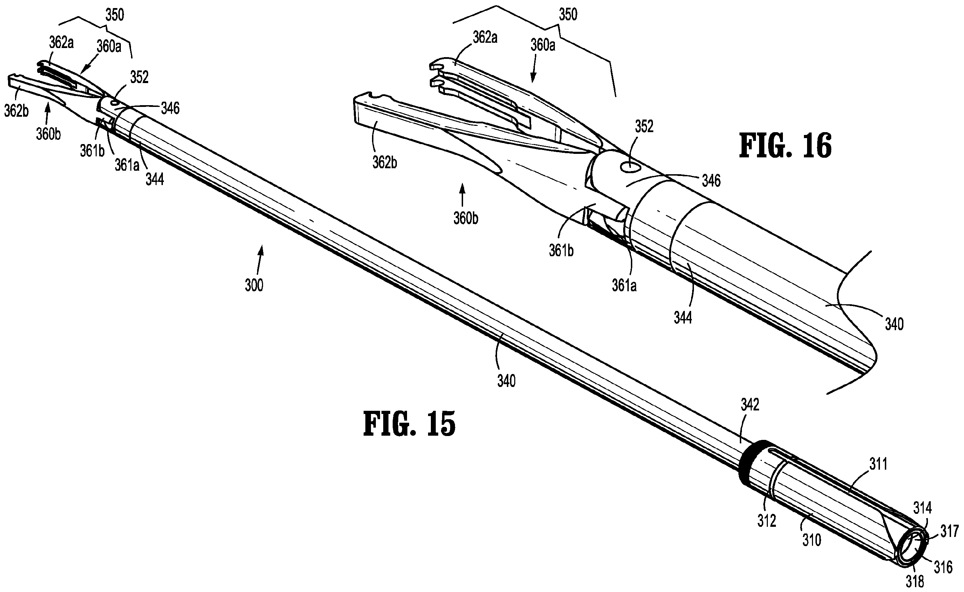

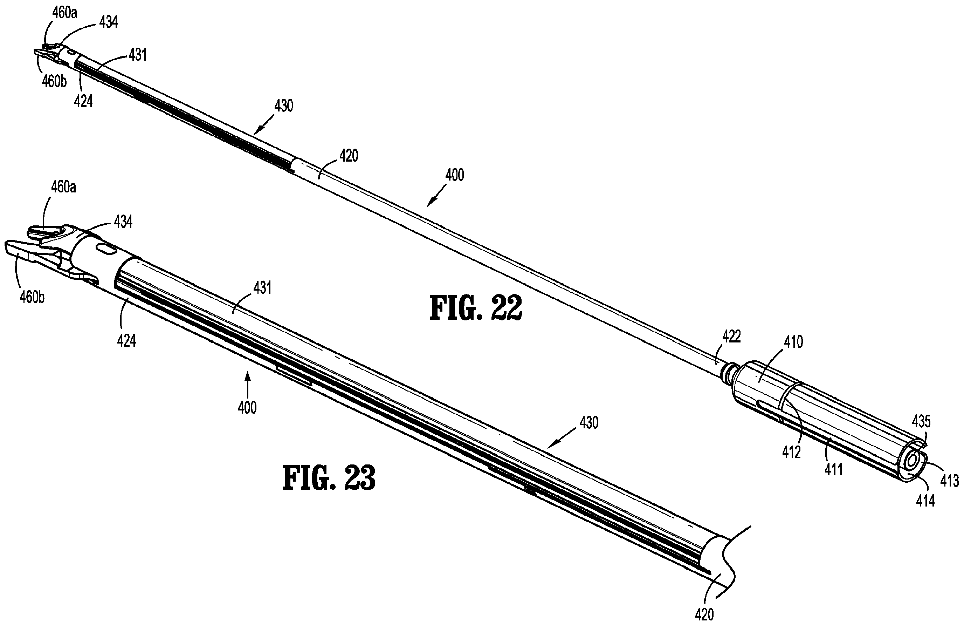

Handle assembly 100 is initially detailed for use in connection with a generic endoscopic assembly 200 that includes features common to any endoscopic assembly usable with handle assembly 100. Exemplary embodiments of particular endoscopic assemblies, e.g., endoscopic assembly 300 (FIG. 15) and endoscopic assembly 400 (FIG. 22), are thereafter detailed below. Endoscopic assembly 300 (FIG. 15), for example, is configured for grasping and manipulating tissue, retrieving a surgical clip, and firing and forming the surgical clip about tissue. Endoscopic assembly 400 (FIG. 22), as another example, includes at least one surgical clip loaded therein and is configured to sequentially fire and form the at least one surgical clip about tissue. It is also envisioned that various other endoscopic assemblies for performing various different surgical tasks and/or having various different configurations may be provided for use with handle assembly 100.

Continuing with reference to FIGS. 1 and 2, as noted above, endoscopic assembly 200 is configured to selectively connect to and extend distally from handle assembly 100. Endoscopic assembly 200 includes a proximal hub 210 configured for insertion into and releasable engagement within handle assembly 100, an elongated shaft 220 extending distally from proximal hub 210, and an end effector assembly (not shown) disposed at the distal end of elongated shaft 220. Internal drive components (not shown) extend through proximal hub 210 and elongated shaft 220 so as to operably couple the end effector assembly (not shown) with handle assembly 100 upon engagement of endoscopic assembly 200 with handle assembly 100, e.g., to enable performing the one or more surgical tasks of the endoscopic assembly 200. Proximal hub 210 defines a generally tubular configuration and includes a longitudinally-extending slot 212 defined therein and an annular groove 214 defined therein. Longitudinally-extending slot 212 defines an open proximal end 213. Annular groove 214 extends circumferentially about proximal hub 210 and intersects longitudinally-extending slot 212, although other non-intersecting configurations are also contemplated.

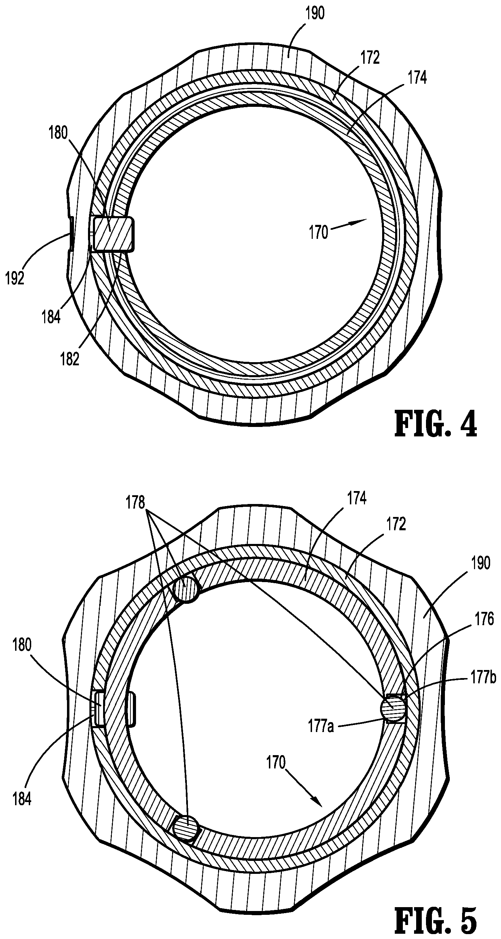

Referring additionally to FIGS. 3-5, handle assembly 100 includes a receiver assembly 170 configured to receive proximal hub 210 of endoscopic assembly 200 and enable releasable engagement of endoscopic assembly 200 with handle assembly 100. Receiver assembly 170 includes an outer collar 172 and an inner tubular member 174. Inner tubular member 174 defines an interior diameter slightly larger than an exterior diameter of proximal hub 210 of endoscopic assembly 200 to enable slidable insertion of proximal hub 210 into inner tubular member 174 without significant play therebetween. Inner tubular member 174 further includes a plurality of apertures 176 defined therethrough and positioned circumferentially about inner tubular member 174. Apertures 176 define reduced interior openings 177a as comparted to the exterior openings 177b thereof. A ball bearing 178 is disposed within each of the apertures 176. Although a portion of each ball bearing 178 protrudes inwardly through the reduced interior opening 177a of its respective aperture 176, the reduced interior openings 177a inhibit ball bearings 178 from passing entirely therethrough. Outer collar 172 is positioned so as to block the exterior openings 177b of apertures, thereby retaining ball bearings 178 within apertures 176 between outer collar 172 and the reduced interior openings 177a (except for the portions of ball bearings 178 extending through the reduced interior openings 177a).

A pin 180 extends through a pin aperture 182 defined within inner tubular member 174 and at least partially through a pin slot 184 defined within outer collar 172. Pin 180 extends at least partially into the interior of inner tubular member 174 and, as detailed below, is configured to facilitate alignment of endoscopic assembly 200 upon insertion of endoscopic assembly 200 into handle assembly 100. Pin 180 is further configured to retain outer collar 172 and inner tubular member 174 in fixed rotational orientation relative to one another. Outer collar 172 is engaged with rotation knob 190 of handle assembly 100 in fixed rotational orientation such that, with pin 180 rotatably coupling outer collar 172 and inner tubular member 174, rotation of rotation knob 190 can be effected to similarly rotate receiver assembly 170. Rotation knob 190 includes an alignment indicator 192 disposed thereon that is aligned with pin 180 to enable alignment of endoscopic assembly 200 with receiver assembly 170 without the need to directly view the position of pin 180.

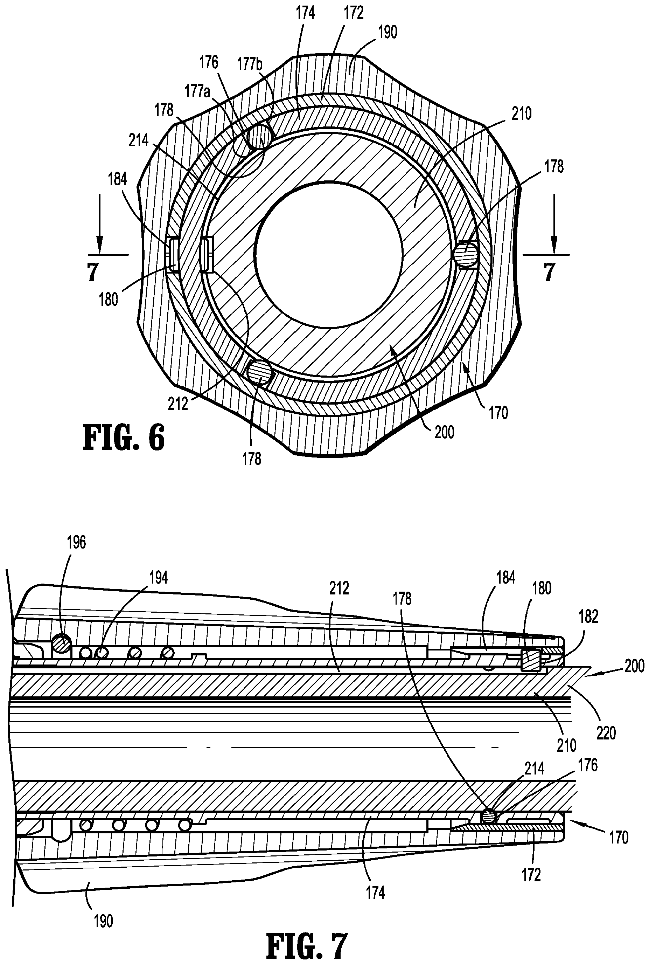

With reference to FIGS. 1, 2, 6 and 7, in order to engage endoscopic assembly 200 with handle assembly 100, endoscopic assembly 200 is oriented such that longitudinally-extending slot 212 thereof is aligned with pin 180 of receiver assembly 170. As noted above, rather than having to view pin 180 directly, alignment of longitudinally-extending slot 212 and pin 180 can be achieved via aligning longitudinally-extending slot 212 with alignment indicator 192 of rotation knob 190 of handle assembly 100. Once alignment has been achieved, proximal hub 210 of endoscopic assembly 200 is slid proximally into inner tubular member 174 of receiver assembly 170. Alignment of longitudinally-extending slot 212 and pin 180 ensures that, upon proximal sliding of proximal hub 210 into inner tubular member 174, pin 180 is translated through longitudinally-extending slot 212.

As proximal hub 210 is slid proximally into inner tubular member 174, ball bearings 178 apply radially-inward force on the exterior of proximal hub 210 causing proximal hub 210, outer collar 172, inner tubular member 174, and/or ball bearings 178 to move or flex to accommodate proximal hub 210 between ball bearings 178. Ball bearings 178 are permitted to rotate within apertures 176 as proximal hub 210 is slid proximally into inner tubular member 174, reducing friction and permitting relatively easy sliding of proximal hub 210 into inner tubular member 174. Upon full insertion of proximal hub 210 into inner tubular member 174, e.g., upon pin 180 reaching the closed, distal end of longitudinally-extending slot 212, ball bearings 178 are moved into position about annular groove 214. As a result of the radially-inward force imparted by ball bearings 178, once the fully inserted position has been achieved, ball bearings 178 are urged into annular groove 214 to thereby releasably lock proximal hub 210 of endoscopic assembly 200 in engagement within receiver assembly 170 of handle assembly 100. The operable coupling of endoscopic assembly 200 with handle assembly 100 to enable operation thereof to perform one or more surgical tasks depends upon the type of endoscopic assembly 200 engaged with handle assembly 100 and will be detailed below with respect to exemplary endoscopic assemblies 300 (FIG. 15) and 400 (FIG. 22).

In order to remove endoscopic assembly 200 from handle assembly 100, endoscopic assembly 200 is pulled distally relative to handle assembly 100 under sufficient urging so as to dislodge ball bearings 178 from annular groove 214, thus permitting proximal hub 210 of endoscopic assembly 200 to be slid distally out of receiver assembly 170 of handle assembly 100.

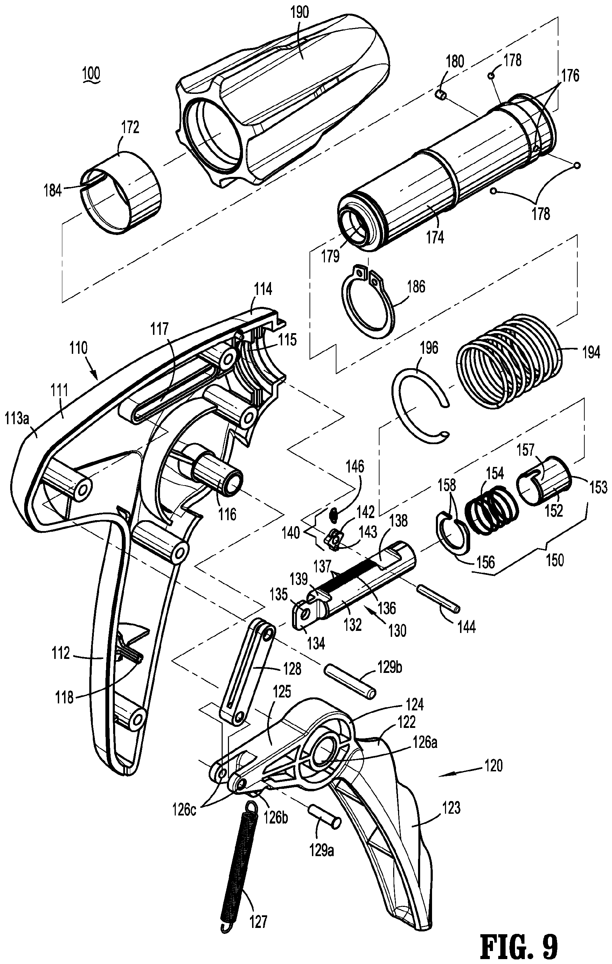

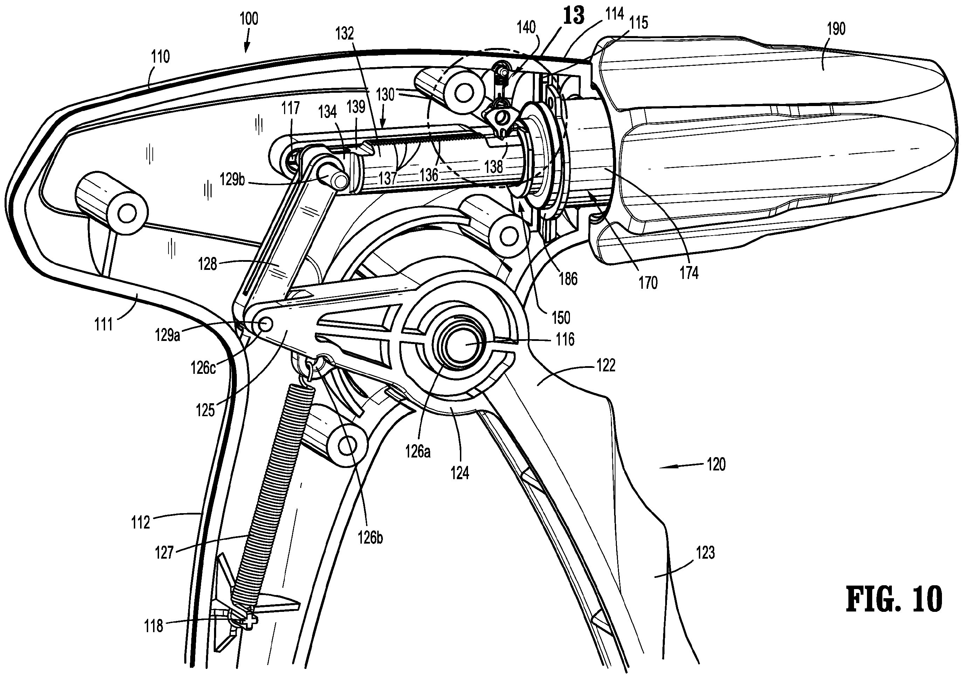

Referring to FIGS. 1, 2, and 8-10, handle assembly 100 generally includes a housing 110, a trigger assembly 120 pivotably coupled to housing 110, a ratcheting drive assembly 130 operably coupled to trigger assembly 120, a bypass assembly 150 operably coupled to ratcheting drive assembly 130, receiver assembly 170 which extends distally from housing 110, and rotation knob 190 which is operably disposed about receiver assembly 170.

Housing 110 defines a body portion 111 and a fixed handle portion 112 extending downwardly from body portion 111. Housing 110 is formed from first and second housing components 113a, 113b secured to one another via pin-post engagement, although first and second housing components 113a, 113b may alternatively be secured in any other suitable manner, e.g., ultrasonic welding, gluing, other mechanical engagement, etc. Housing 110 is configured to house the internal working components of handle assembly 100. Body portion 111 includes a distal nose 114 defining an annular slot 115 on the interior thereof. More specifically, first and second housing components 113a, 113b each define a semi-annular slot portion such that, when first and second housing components 113a, 113b cooperate to form housing 110, annular slot 115 is formed. Receiver assembly 170 of handle assembly 100 includes a retention clip 186 disposed about the proximal end of inner tubular member 174 thereof. Retention clip 186 is captured within annular slot 115 defined within distal nose 114 of housing 110, e.g., upon engagement of first and second housing components 113a, 113b with one another. Retention clip 186 is captured within annular slot 115 to rotatably engage receiver assembly 170 with housing 110. Rotation knob 190 of handle assembly 100 is operably engaged about receiver assembly 170, e.g., via outer collar 172, biasing member 194, and elastomeric C-ring 196, in fixed rotational orientation relative thereto such that rotation of rotation knob 190 relative to housing 110 effects similar rotation of receiver assembly 170 relative to housing 110. Thus, with endoscopic assembly 200 engaged within receiver assembly 170, rotation knob 190 may be rotated relative to housing 100 to similarly rotate endoscopic assembly 200 relative to housing 110.

Body portion 111 of housing 110 further incudes an internal pivot post 116 extending transversely between housing components 113a, 113b and a longitudinally-extending guide track 117 defined within one or both of housing components 113a, 113b, the importance of each of which is detailed below. Fixed handle portion 112 of housing 110 is configured to facilitate grasping of handle assembly 100 and manipulation thereof and is monolithically formed with body portion 111, although other configurations are also contemplated.

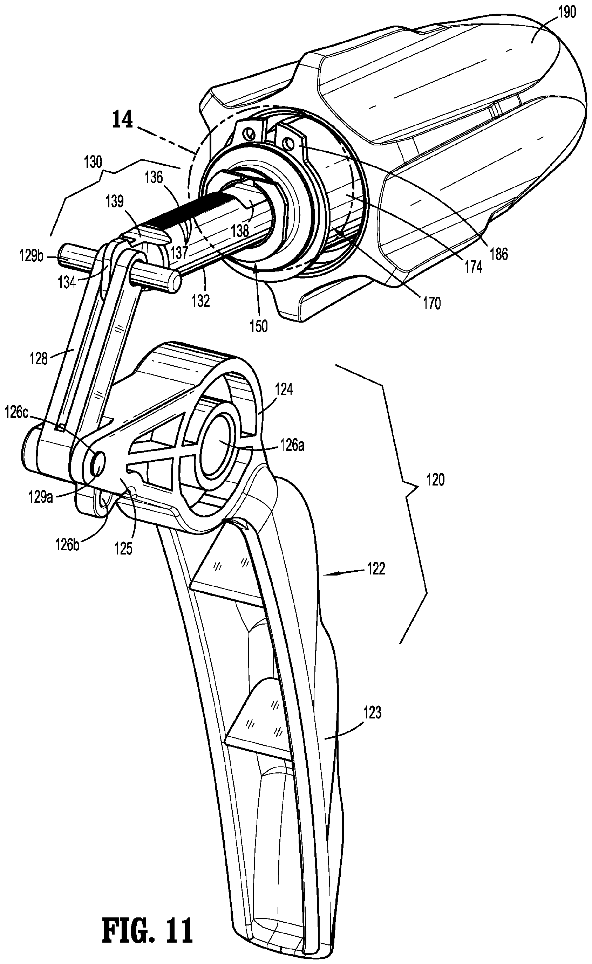

With additional reference to FIG. 11, trigger assembly 120 generally includes a trigger 122, a biasing member 127, and a linkage 128. Trigger 122 includes a grasping portion 123, an intermediate pivot portion 124, and a proximal extension portion 125. Grasping portion 123 of trigger 122 extend downwardly from body portion 111 of housing 110 in opposed relation relative to fixed handle portion 112 of housing 110. Grasping portion 123 is configured to facilitate grasping and manipulation of trigger 122. Intermediate pivot portion 124 of trigger 122 is at least partially disposed within housing 110 and defines a pivot aperture 126a that is configured to receive pivot post 116 of housing 110 so as to enable pivoting of trigger 122 about pivot post 116 and relative to housing 110, e.g., between an un-actuated position, wherein grasping portion 123 of trigger 122 is spaced-apart relative to fixed handle portion 112, and an actuated position, wherein grasping portion 123 of trigger 122 is approximated relative to fixed handle portion 112.

Proximal extension portion 125 of trigger 122 of trigger assembly 120 is disposed on an opposite side of intermediate pivot portion 124 and, thus, pivot post 116, as compared to grasping portion 123 of trigger 122. As such, pivoting of grasping portion 123 proximally, e.g., towards the actuated position, urges proximal extension portion 125 distally. Proximal extension portion 125 includes a first aperture 126b configured to receive a first end of biasing member 127, and a pair of second apertures 126c configured to receive a first pin 129a for pivotably coupling the proximal end of linkage 128 and proximal extension portion 125 of trigger 122 with each other. The second end of biasing member 127 is engaged about an arm 118 extending transversely within fixed handle portion 112. Biasing member 127 is disposed in an at-rest condition in the un-actuated position of grasping portion 123 of trigger 122. Pivoting of grasping portion 123 towards the actuated position elongates biasing member 127 storing energy therein such that, upon release of grasping portion 123, grasping portion 123 is returned towards the un-actuated position under the bias of biasing member 127. Although illustrated as an extension coil spring, biasing member 127 may define any suitable configuration for biasing grasping portion 123 of trigger 122 towards the un-actuated position.

As noted above, linkage 128 is coupled at its proximal end to proximal extension portion 125 of trigger 122 via first pin 129a. Linkage 128 is also pivotably coupled, at its distal end, to proximal extension 134 of drive bar 132 of ratcheting drive assembly 130 via a second pin 129b. Second pin 129b extends outwardly from either or both sides of proximal extension 134 of drive bar 132 and is received within the longitudinally-extending guide track(s) 117 defined within housing component 113a and/or housing component 113b. As a result of this configuration, pivoting of grasping portion 123 towards the actuated position urges proximal extension portion 125 distally which, in turn, urges linkage 128 distally such that second pin 129b is translated distally through longitudinally-extending guide track(s) 117.

Continuing with reference to FIGS. 1, 2, and 8-11, ratcheting drive assembly 130 of handle assembly 100 includes a drive bar 132 and a pawl assembly 140. Drive bar 132 includes a proximal extension 134, a ratchet rack 136, and distal and proximal recesses 138, 139, respectively. Proximal extension 134 is disposed at the proximal end of the drive bar 132 and defines an aperture 135 configured to receive second pin 129b of trigger assembly 120 so as to pivotably couple the distal end of linkage 128 and drive bar 132 with one another, as noted above. As such, upon pivoting of grasping portion 123 towards the actuated position to urge second pin 129b distally through longitudinally-extending guide track(s) 117, drive bar 132 is translated distally through body portion 111 of housing 110. Ratchet rack 136 of drive bar 132 defines a plurality of teeth 137 and extends longitudinally along drive bar 132 on an upper surface thereof. Distal and proximal recesses 138, 139 are defined by cut-outs formed in drive bar 132 and are positioned distally adjacent ratchet rack 136 and proximally adjacent ratchet rack 136, respectively.

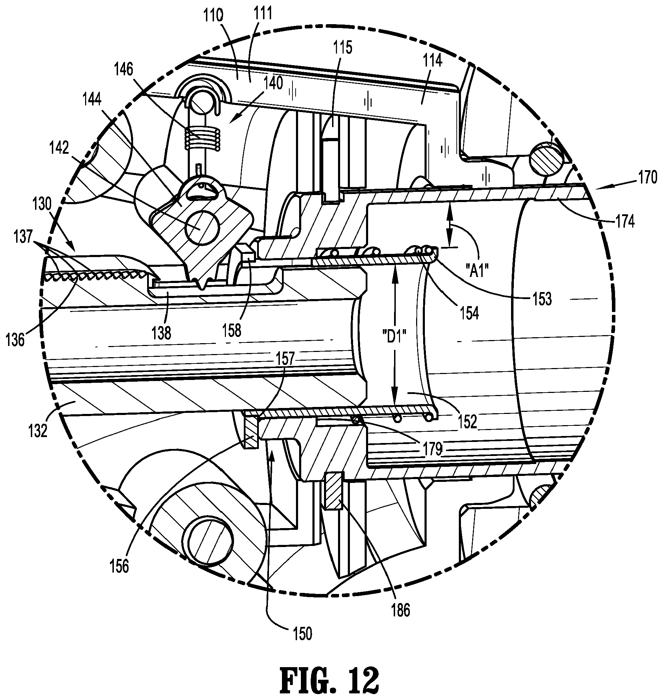

Referring also to FIG. 12, pawl assembly 140 of ratcheting drive assembly 130 includes a ratchet pawl 142, a pawl pin 144, and a pawl biasing member 146. Ratchet pawl 142 is pivotably coupled to body portion 111 of housing 110 by pawl pin 144 so as to enable operable engagement of ratchet pawl 142 with ratchet rack 136 when an endoscopic assembly 200 that uses the ratcheting function is connected to handle assembly 100, and to enable pivoting of ratchet pawl 142 to a bypass position when a endoscopic assembly 200 that does not use the ratcheting function is connected to handle assembly 100. Ratchet pawl 142 further includes a pair of outwardly-extending tabs 143 extending transversely from either side thereof, the importance of which are detailed below.

Pawl biasing member 146 of pawl assembly 140 is coupled between ratchet pawl 142 and body portion 111 of housing 110 so as to bias ratchet pawl 142 towards a use position and away from the bypass position. In the use position, ratchet pawl 142 is oriented to operably engage ratchet rack 136 upon distal advancement of drive bar 132. However, in the proximal-most position of drive bar 132, corresponding to the un-actuated position of trigger 122, ratchet pawl 142 is disposed at least partially within distal recess 138 of drive bar 132. Accordingly, at least initially, ratchet pawl 142 is disengaged from ratchet rack 136.

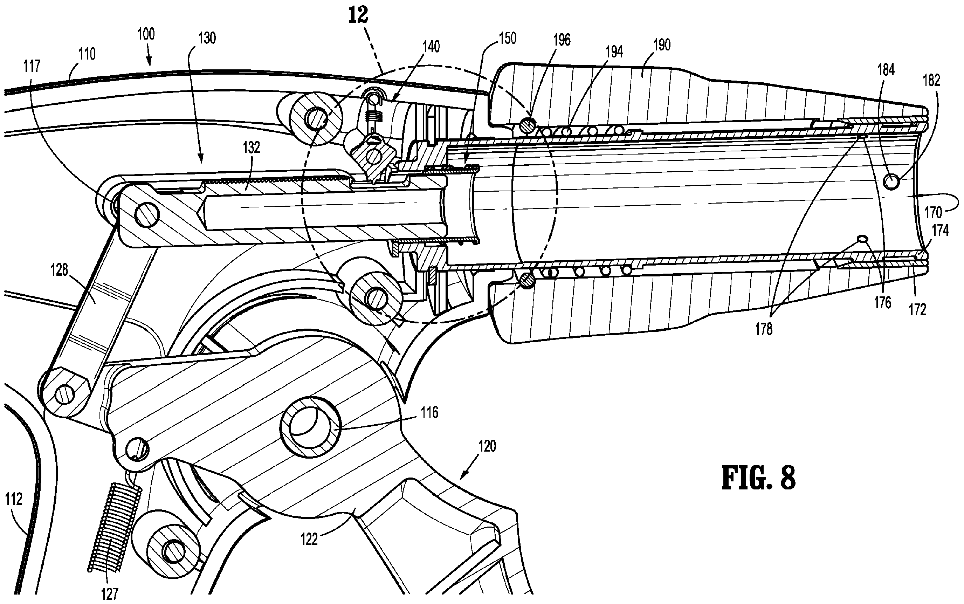

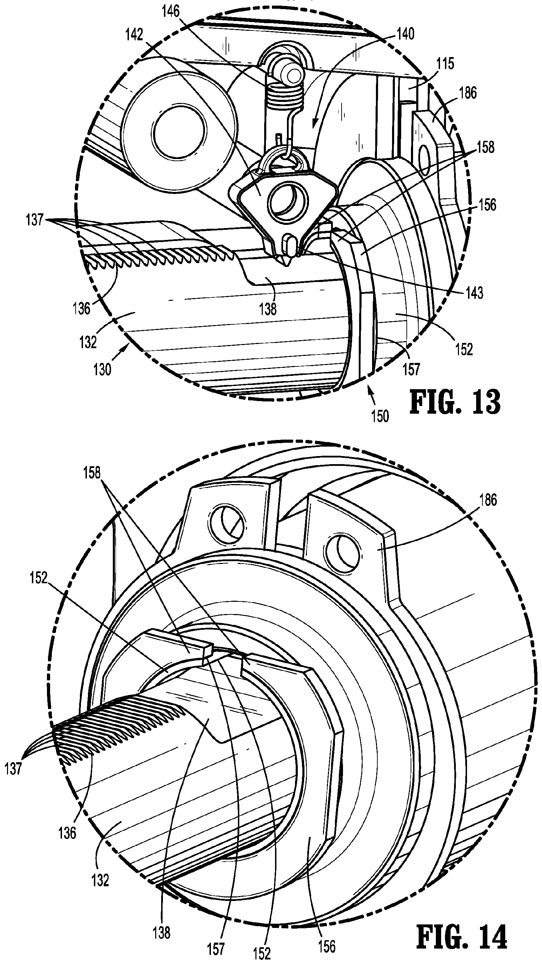

With reference to FIGS. 8-14, bypass assembly 150 is operably positioned between pawl assembly 140 and receiver assembly 170 and is configured, in response to engagement of handle assembly 100 with an endoscopic assembly 200 that does not use the ratcheting function, to pivot ratchet pawl 142 to the bypass position, thereby inhibiting ratcheting upon advancement of drive bar 132. When an endoscopic assembly 200 that uses the ratcheting function is connected to handle assembly 100, bypass assembly 150 remains idle such that ratchet pawl 142 remains in the use position to enable ratcheting of ratchet pawl 142 along ratchet rack 136 upon advancement of drive bar 132.

Bypass assembly 150 includes a sleeve 152, a biasing member 154, and a camming clip 156. Sleeve 152 extends into the proximal end of inner tubular member 174 of receiver assembly 170 and is disposed about the distal end of drive bar 132 of drive assembly 130 in slidable relation relative to both inner tubular member 174 and drive bar 132. Biasing member 154 is disposed within inner tubular member 174 of receiver assembly 170 and about sleeve 152. More specifically, biasing member 154 is retained about sleeve 152 between a distal rim 153 of sleeve 152 and an annular shoulder 179 defined within the interior of inner tubular member 174 at the proximal end thereof. As a result of this configuration, biasing member 154 biases sleeve 152 proximally into the interior of inner tubular member 174. Distal rim 153 of sleeve 152 is radially-spaced from the interior wall defining inner tubular member 174 so as to define an annular spacing "A1" therebetween. Sleeve 152 further defines an internal diameter "D1."

Camming clip 156 of bypass assembly 150 is engaged within an annular groove 157 defined about the exterior of sleeve 152 towards the proximal end thereof. Camming clip 156 is sufficiently dimensioned so as to inhibit passage into the interior of inner tubular member 174 and, thus, inhibits sleeve 152 from fully entering inner tubular member 174 under the bias of biasing member 154. Camming clip 156 further include a pair of opposed, inwardly extending fingers 158 at the free ends thereof. Fingers 158 are positioned such that, upon sufficient proximal urging of sleeve 152 against the bias of biasing member 154, fingers 158 contact respective tabs 143 of ratchet pawl 142. Thus, upon further proximal movement of sleeve 152, fingers 158 urge respective tabs 143 proximally, ultimately such that ratchet pawl 142 is urged to rotate about pawl pin 144 and against the bias of pawl biasing member 146 from the use position to the bypass position.

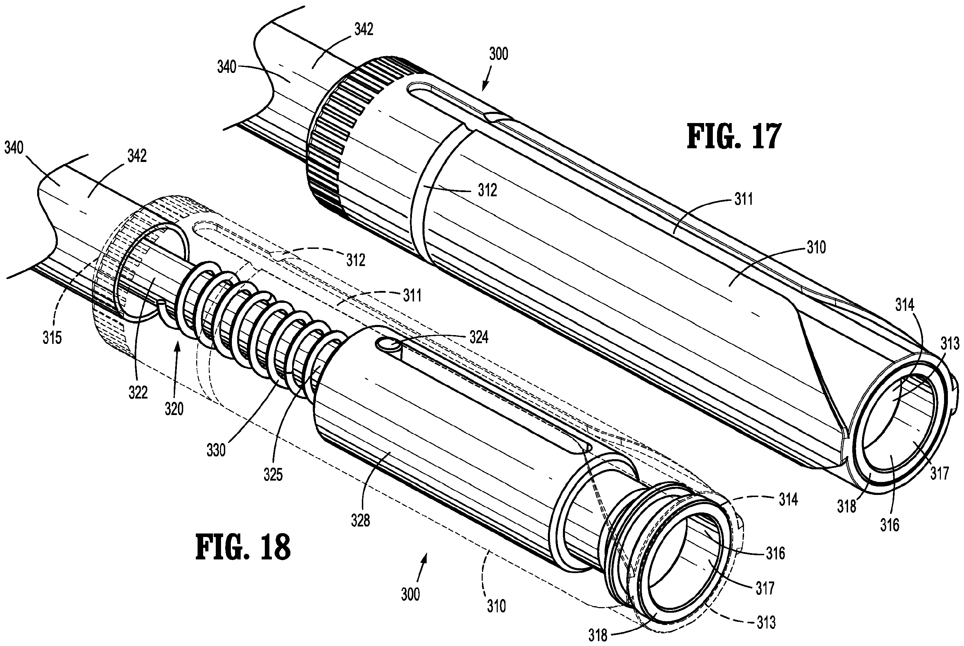

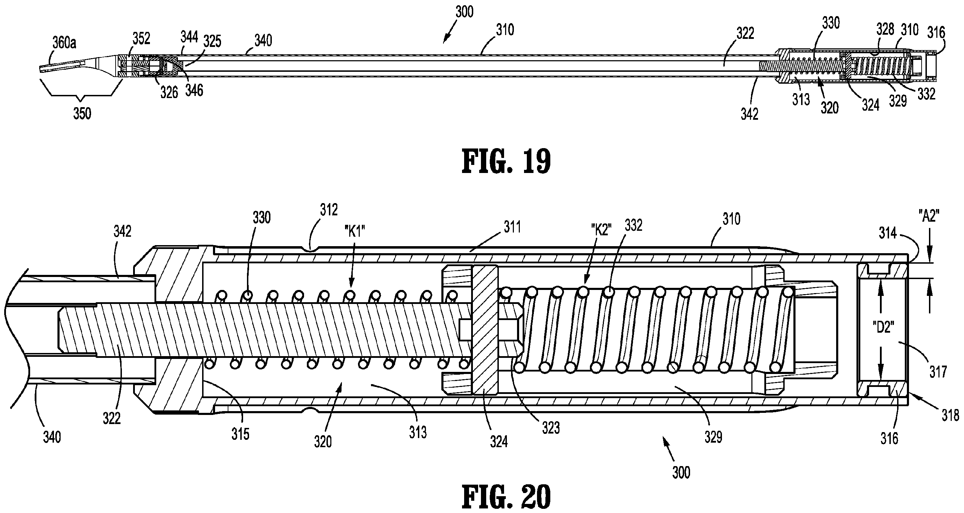

Turning to FIGS. 15-21, and endoscopic assembly 300 provided in accordance with the present disclosure and configured for use with handle assembly 100 is shown. Endoscopic assembly 300 is configured for non-ratcheting use and, thus, upon engagement of endoscopic assembly 300 with handle assembly 100, as detailed below, ratchet pawl 142 is pivoted to and retained in the bypass position, thus enabling such non-ratcheting use. Endoscopic assembly 300 generally includes a proximal hub 310, an inner drive assembly 320 disposed within and extending through proximal hub 310, an elongated shaft 340 extending distally from proximal hub 310, and an end effector assembly 350 including a pair of jaw members 360a, 360b disposed at the distal end of elongated shaft 340. Endoscopic assembly 300 is configured to grasp and/or manipulate tissue, retrieve a surgical clip, and to close, fire, or form the surgical clip about tissue. It is contemplated that endoscopic assembly 300 be configured to close, fire or form surgical clips similar to those shown and described in U.S. Patent No. 4,834,096, the entire contents of which are incorporated herein by reference.

With additional reference to FIGS. 1, 2, 6, and 7, proximal hub 310 of endoscopic assembly 300 defines a generally tubular configuration and an exterior diameter slightly smaller than that of inner tubular member 174 of receiver assembly 170 of handle assembly 100 to enable slidable insertion of proximal hub 310 into inner tubular member 174 without significant play therebetween. Proximal hub 310 further includes features similar to those detailed above with respect to endoscopic assembly 200 so as to enable engagement of proximal hub 310 within receiver assembly 170 of handle assembly 100 in a similar fashion. More specifically, proximal hub 310 a longitudinally-extending slot 311 configured to receive pin 180 of receiver assembly 170 to ensure proper alignment of endoscopic assembly 300 relative to handle assembly 100, and an annular groove 312 configured to receive at least a portion of each ball bearing 178 to releasably lock proximal hub 310 of endoscopic assembly 300 in engagement within receiver assembly 170 of handle assembly 100.

Referring again to FIGS. 15-21, proximal hub 310 of endoscopic assembly 300 further defines an internal bore 313 having an open proximal end 314 and a reduced-diameter distal opening as compared to the diameter of bore 313 so as to define a shoulder 315 therebetween. A ferrule 316 is seated within the open proximal end of proximal hub 310 and secured therein in any suitable fashion, e.g., welding, gluing, press-fitting, mechanical engagement, etc.

Ferrule 316 of proximal hub 310 defines an aperture 317 extending longitudinally therethrough and a proximally-facing surface 318 surrounding aperture 317 such that proximally-facing surface 318 defines a ring-shaped configuration. Aperture 317 is disposed in communication with the interior of proximal hub 310 so as to provide access to inner drive assembly 320, as detailed below, and defines a diameter "D2" that is sufficiently large so as to permit slidable insertion of drive bar 132 of ratcheting drive assembly 130 of handle assembly 100 therethrough. However, diameter "D2" of aperture 317 is smaller than internal diameter "D1" of sleeve 152. Proximally-facing surface 318 of ferrule 316 defines an annular width "A2" that is larger than the annular spacing "A1" defined between distal rim 153 of sleeve 152 and the interior wall defining inner tubular member 174. As a result of diameter "D2" being smaller than diameter "D1" and annular width "A2" being larger than annular spacing "A1," proximal hub 310 is inhibited from passing into the interior of sleeve 152 and is likewise inhibited from passing about the exterior of sleeve 152. Rather, upon proximal urging of proximal hub 310 of endoscopic assembly 300 into inner tubular member 174 of receiver assembly 170 of handle assembly 100, e.g., to engage endoscopic assembly 300 with handle assembly 100, proximally-facing surface 318 of ferrule 316 eventually contacts distal rim 153 of sleeve 152 such that further proximal urging of proximal hub 310 into inner tubular member 174 urges sleeve 152 proximally against the bias of biasing member 154.

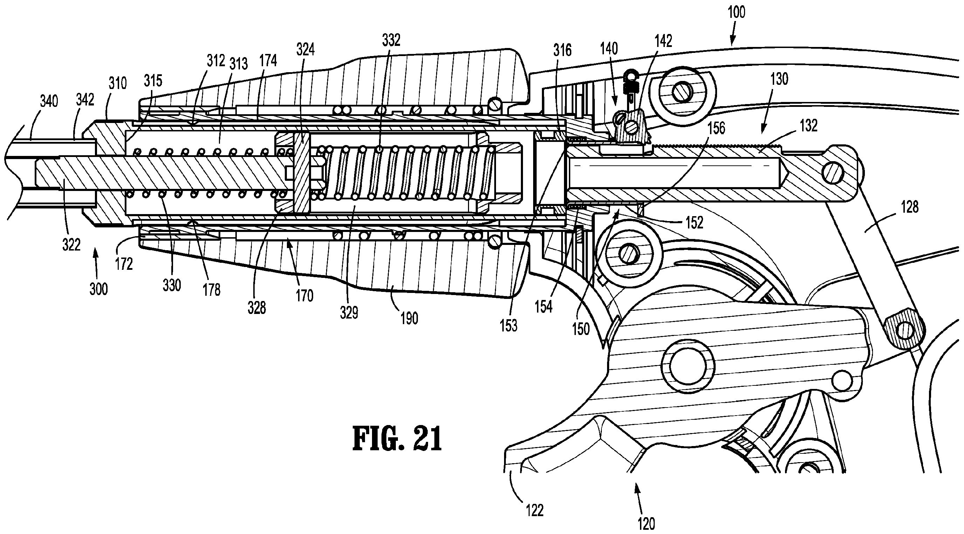

As noted above, endoscopic assembly 300 is configured for non-ratcheting use. Accordingly, the above-detailed configuration regarding the relative dimensions of the components of proximal hub 310 and those of bypass assembly 150 ensures that proximal hub 310 urges ratchet pawl 142 from the use position to the bypass position upon engagement of endoscopic assembly 300 with handle assembly 100, thus disabling the ratcheting components of ratcheting drive assembly 130. More specifically, with pin 180 received within longitudinally-extending slot 311 and proximal hub 310 sliding proximally into inner tubular member 174 of receiver assembly 170, but prior to engagement of ball bearings 178 within annular groove 312, proximally-facing surface 318 of ferrule 316 contacts distal rim 153 of sleeve 152 and urges sleeve 152 proximally such that fingers 158 of camming clip 156 urge tabs 143 of ratchet pawl 142 proximally to thereby rotate ratchet pawl 142 about pawl pin 144 from the use position towards the bypass position. Accordingly, upon reaching the engaged position of proximal hub 310 within inner tubular member 174, e.g., upon engagement of ball bearings 178 within annular groove 312, as shown in FIG. 21, ferrule 316 has urged sleeve 152 to a proximal-most position wherein ratchet pawl 142 is pivoted to and retained in the bypass position. Thus, when endoscopic assembly 300 is engaged with handle assembly 100, ratcheting of ratcheting drive assembly 130 is disabled.

Referring still to FIGS. 15-21, inner drive assembly 320 of endoscopic assembly 300 includes an inner shaft 322 slidably disposed within both proximal hub 310 and elongated shaft 340 of endoscopic assembly 300. Inner shaft 322 includes a proximal end 323 supporting a transverse pin 324 disposed within bore 313 of proximal hub 310, and a distal end 325 supporting a cam pin 326 disposed towards the distal end 344 of elongated shaft 340. As detailed below, cam pin 326 is disposed within cam slots (not shown) of jaw members 360a, 360b of end effector assembly 350 to enable pivoting of jaw members 360a, 360b between open and closed positions in response to translation of inner shaft 322 through elongated shaft 340.

Inner drive assembly 320 further includes a plunger 328 and first and second biasing members 330, 332, respectively. Plunger 328 is slidably disposed within bore 313 of proximal hub 310 and is retained therein between shoulder 315 and ferrule 316. Plunger 328 defines an internal cavity 329 within which transverse pin 324 of proximal end 323 of inner shaft 322 is slidably confined.

First biasing member 330 of inner drive assembly 320 is disposed within internal bore 313 of proximal hub 310 and interposed between shoulder 315 of proximal hub 310 and transverse pin 324 of inner shaft 322. First biasing member 330 has a first spring constant "K1" which is less than a second spring constant "K2" of second biasing member 332, the importance of which is detailed below. Second biasing member 332 is disposed within cavity 329 of plunger 328 and is interdisposed between transverse pin 324 of inner shaft 322 and the proximal end of plunger 328. As detailed below, first and second biasing members 330, 332, respectively, facilitate appropriate translation of inner shaft 322 through proximal hub 310 and elongated shaft 340 to open and close jaw members 340a, 340b, and to enable full actuation of trigger 122 (FIG. 1), as detailed below.

Elongated shaft 340 of endoscopic assembly 300 defines a generally tubular configuration and extends between and interconnects proximal hub 310 and end effector assembly 350. More specifically, the proximal end 342 of elongated shaft 340 is secured to proximal hub 310, while the distal end 344 of elongated shaft 340 supports a clevis 346 configured to pivotably engage jaw members 360a, 360b of end effector assembly 350 at distal end 344 of elongated shaft 340 via a pivot pin 352.

End effector assembly 350, as noted above, includes first and second jaw members 360a, 360b. Jaw members 360a, 360b are pivotably engaged to one another and clevis 346 via pivot pin 352 so as to permit pivoting of jaw members 360a, 360b relative to one another and elongated shaft 340 between an open position and a closed position. Each jaw member 360a, 360b includes a respective proximal end 361a, 361b and a respective distal end 362a, 362b. The proximal end 361a, 361b of each jaw member 360a, 360b defines the cam slots (not shown) that are configured to receive cam pin 326 of inner shaft 322 such that translation of inner shaft 322 pivots jaw members 360a, 360b between the open and closed positions. The distal ends 362a, 362b of jaw members 360a, 360b are configured to receive and close, fire or form a surgical clip, e.g., a surgical clip similar to those shown and described in U.S. Patent No. 4,834,096, previously incorporated herein by reference.

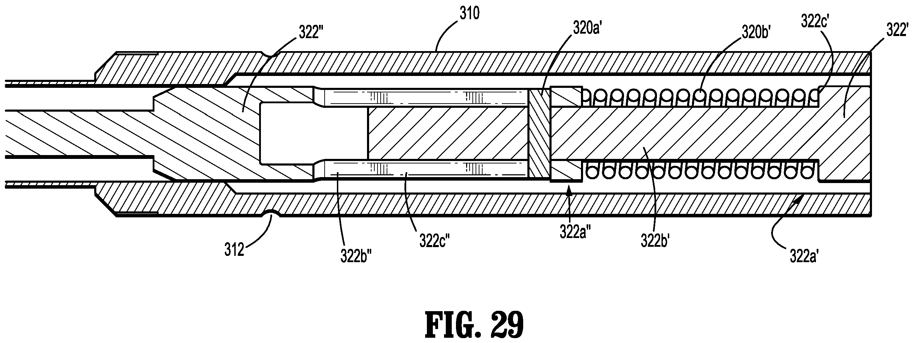

Referring momentarily to FIG. 29, an alternate embodiment of inner drive assembly 320 is illustrated. In this embodiment, inner shaft 322 of endoscopic assembly 300 is divided into a proximal portion 322' and a distal portion 322". A proximal end 322a" of distal portion 322" includes a bore 322b" defined therein configured to slidably receive an elongate member 322b' disposed on a distal end 322a' of proximal portion 322'. A transverse slot 322c" is defined through distal portion 322" of inner shaft 322 and is configured to slidably retain a transverse pin 320a'. Transverse pin 320a' is fixedly retained within an aperture (not shown) defined in the distal end 322a' of proximal portion 322' using any suitable means, such as friction fit, welding, adhesives, or the like. A biasing member 320b' is disposed between proximal portion 322' and distal portion 322" of inner shaft 322 and acts upon proximal end 322a" of distal portion 322" and an annular surface 322c' disposed on a distal end 322a' of proximal portion 322'. In this manner, biasing member (e.g., a spring or the like) 320b' is initially compressed such that proximal portion 322' and distal portion 322" are maintained in spaced relation. Transverse pin 320a' inhibits proximal portion 322' and distal portion 322" from being urged apart by biasing member 320b' as transverse pin 320b' is at a proximal most position in the stroke of transverse slot 322c".

In operation, if the closure of the jaw members 360a, 360b should become stuck or otherwise prevented from closing completely (e.g., where the jaw members 360a, 360b are closing onto bone or onto another surgical clip), this over-load compensation system permits a forward stroke of ratcheting drive assembly 130 of handle assembly 100 may be fully completed (wherein a distal driving force of proximal portion 322' of inner shaft 322 axially compresses biasing member 320b' having a spring constant "K3", which is greater than that of "K1" or "K2") in order to permit a re-set or a reversal of ratcheting drive assembly 130 and permit trigger 122 to open.

The use of handle assembly 100 in conjunction with endoscopic assembly 300 is now detailed with reference to FIGS. 8-21. Initially, endoscopic assembly 300 is engaged with handle assembly 100, as detailed above. Such engagement of endoscopic assembly 300 with handle assembly 100, as also detailed above, effects pivoting of ratchet pawl 142 to and retention of ratchet pawl 142 in the bypass position. Once endoscopic assembly 300 and handle assembly 100 are engaged with ratchet pawl 142 in the bypass position, handle assembly 100 and endoscopic assembly 300 are together ready for use.

In use, trigger 122 is initially disposed in the un-actuated position under the bias of biasing member 127. With trigger 122 disposed in the un-actuated position, drive bar 132 is disposed in a proximal-most position. Further, inner shaft 322 is disposed in a proximal-most position under the bias of first and second biasing members 330, 332. Thus, jaw members 360a, 360b, initially, are disposed in the open position. With jaw members 360a, 360b disposed in the open position, a new, unformed or open surgical clip (not shown) may be located or loaded within the distal ends 362a, 362b of jaw members 360a, 360b. Jaw members 360a, 360b of end effector assembly 350 may be used to retrieve or pick-up a surgical clip from a clip holder (not shown), the surgical clip may be manually loaded by the user, end effector assembly 350 may be pre-loaded by the manufacturer, or the surgical clip may be placed between jaw members 360a, 360b in any other suitable fashion.

In or to close, fire, or form the surgical clip loaded between jaw members 360a, 360b, trigger 122 is urged from the un-actuated position to the actuated position. More specifically, grasping portion 123 of trigger 122 is pivoted towards fixed handle portion 112 of housing 110 to urge linkage 128 distally which, in turn, urges drive bar 132 distally through housing 110, receiver assembly 170, and into bore 313 of proximal hub 310 of endoscopic assembly 300. As trigger 122 is pivoted further towards the actuated position, drive bar 132 eventually contacts plunger 328 of drive assembly 320 of endoscopic assembly 300. Due to first spring constant "K1" of first biasing member 330 being less than second spring constant "K2" of second biasing member 332, as drive bar 132 is initially urged into plunger 328, plunger 328 and inner shaft 322 translate together distally such that first biasing member 330 is compressed while second biasing member 332 remains substantially un-compressed.

As inner shaft 322 is translated distally, cam pin 326 is translated through the cam slots of jaw members 360a, 360b to pivot jaw members 360a, 360b towards the closed position to close and/or form the surgical clip (not shown) loaded within end effector assembly 350. Cam pin 326 is advanced distally until cam pin 326 reaches an end of the cam slots of jaw members 360a, 360b and/or until jaw members 360a, 360b are fully approximated against one another or fully closed on the surgical clip. As can be appreciated, depending upon the particular endoscopic assembly used, the configuration of the surgical clip being formed, and/or other factors, the required travel distance of inner shaft 322 to fully form the surgical clip may vary. As the distance of travel for trigger 122 between the un-actuated and actuated positions does not vary, it is endoscopic assembly 300 that accounts for this variation, as detailed below.

Once jaw members 360a, 360b have been fully approximated against one another or fully closed on the surgical clip, and/or when cam pin 326 has reached the end of the cam slots of jaw members 360a, 360b, inner shaft 322 is no longer permitted to travel further distally. Thus, upon further distal urging of drive bar 132, e.g., to complete the actuation stroke of trigger 122, plunger 328 is advanced distally independently of inner shaft 322 to compress second biasing member 332. Thus, the compression of second biasing member 332 enables inner shaft 322 to remain in position while the full actuation stroke of trigger 122 is completed.

Once the surgical clip has been fully formed, trigger 122 may be released and allowed to return under bias to the un-actuated position, thereby pulling drive bar 132 back to its proximal-most position and allowing jaw members 360a, 360b to return to the open position. Thereafter, the above-detailed use may be repeated to close, fire, or form additional surgical clips. Additionally or alternatively, jaw members 360a, 360b of end effector assembly 350 may be used to grasp and/or manipulate tissue as desired prior to or after formation of one or more surgical clips.

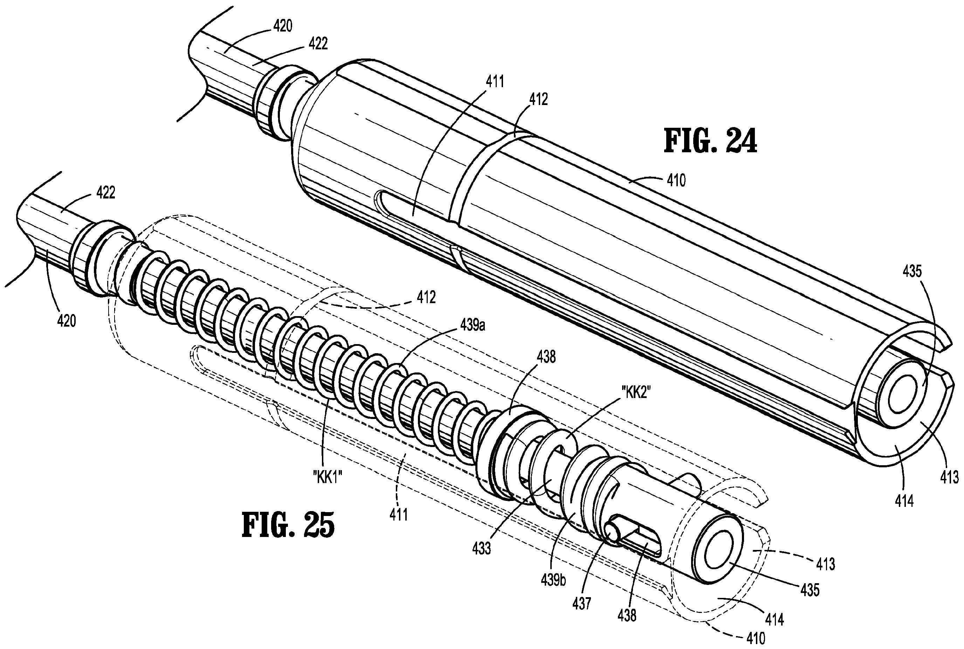

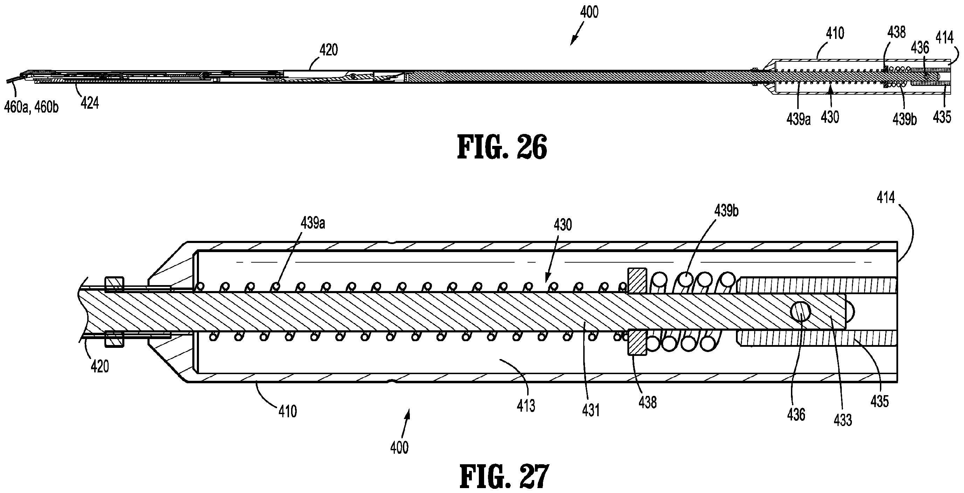

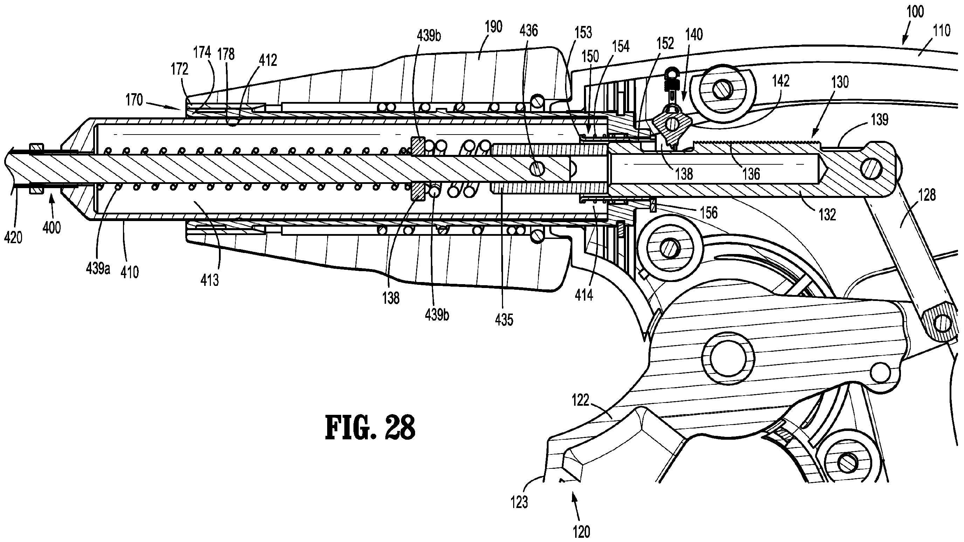

Turning to FIGS. 22-28, another endoscopic assembly 400 provided in accordance with the present disclosure and configured for use with handle assembly 100 (FIG. 1) is shown. Endoscopic assembly 400 is configured for ratcheting use and, thus, upon engagement of endoscopic assembly 400 with handle assembly 100, as detailed below, ratchet pawl 142 remains in the use position to enable ratcheting use. Endoscopic assembly 400 generally includes a proximal hub 410, an elongated shaft 420 extending distally from proximal hub 410, a drive assembly 430 disposed within proximal hub 410 and elongated shaft 420, and a pair of jaw members 460a, 460b supported at the distal end of elongated shaft 420. Endoscopic assembly 400 is configured to close, fire, or form one or more surgical clips about tissue. More specifically, it is contemplated that endoscopic assembly 400 may be configured to close, fire or form surgical clips similar to those shown and described in U.S. Patent Nos. 7,819,886 or 7,905,890, the entire contents of each of which is incorporated herein by reference.

With reference also to FIGS. 1, 2, 6, and 7, proximal hub 410 further includes features similar to those detailed above with respect to endoscopic assembly 200 so as to enable engagement of proximal hub 410 within receiver assembly 170 of handle assembly 100 in a similar fashion. More specifically, proximal hub 410 a longitudinally-extending slot 411 configured to receive pin 180 of receiver assembly 170 to ensure proper alignment of endoscopic assembly 400 relative to handle assembly 100, and an annular groove 412 configured to receive at least a portion of each ball bearing 178 to releasably lock proximal hub 410 of endoscopic assembly 400 in engagement within receiver assembly 170 of handle assembly 100.

As noted above, endoscopic assembly 400 is configured for ratcheting use and, thus, upon engagement of endoscopic assembly 400 with handle assembly 100 ratchet pawl 142 remains in the use position to enable ratcheting use. To allow such, proximal hub 410 defines a ring-shaped aperture 414 annularly disposed between the outer housing defining proximal hub 410 and plunger 435 of drive assembly 430, which is slidably disposed within proximal hub 410. This ring-shaped aperture 414 is positioned and dimensioned to receive distal rim 153 of sleeve 152 upon insertion of endoscopic assembly 400 into receiver assembly 170. Thus, upon insertion of proximal hub 410 of endoscopic assembly 400 into inner tubular member 174 of receiver assembly 170 of handle assembly 100, e.g., to engage endoscopic assembly 400 with handle assembly 100, distal rim 153 of sleeve 152 passes into proximal hub 410 through ring-shaped aperture 414 undisturbed such that sleeve 152 is maintained in its distal-most position under the bias of biasing member 154. With sleeve 152 in its distal-most position, ratchet pawl 142 remains in the use position, thus enabling ratcheting use of ratcheting drive assembly 130 of handle assembly 100.

Referring back to FIGS. 22-28, as mentioned above, endoscopic assembly 400 includes an elongated shaft 420 extending distally from proximal hub 410. Elongated shaft 420 includes a proximal end 422 secured to proximal hub 410 and a distal end 424 supporting first and second jaw members 460a, 460b.

Drive assembly 430 includes an inner shaft 431 slidably supported within the interior of elongated shaft 420 and proximal hub 410. Inner shaft 431 includes a proximal end 433 and a distal end 434. The proximal end 433 of inner shaft 431 extends into internal bore 413 of proximal hub 410 and is operably coupled to plunger 435 of drive assembly 430 via receipt of transverse pin 436 of inner shaft 431 within longitudinal slots 437 of plunger 435. Distal end 434 of inner shaft 431 is configured to transition first and second jaw members 460a, 460b from an open position to a closed position to form a surgical clip (not shown) that has been loaded into first and second jaw members 460a, 460b in response to distal translation of inner shaft 431 through elongated shaft 420.

It is contemplated that inner shaft 431 may be split into a proximal portion and a distal portion in a similar manner as disclosed above with respect to inner shaft 322. The components and operation of this embodiment of inner shaft 431 are similar to that of inner shaft 322, and therefore, a detailed description of the components and operation thereof will not be described hereinbelow.

Drive assembly 430 further includes a stop ring 438 and first and second biasing members 439a, 439b, each of which is disposed about inner shaft 431. Stop ring 438 is fixedly engaged about inner shaft 431 and disposed within internal bore 413 of proximal hub 410. First biasing member 439a is positioned distally of stop ring 438 and is retained between stop ring 438 and the distal end of proximal hub 410. Second biasing member 439b is positioned proximally of stop ring 438 and is retained between stop ring 438 and the distal end of plunger 435. First biasing member 439a has a first spring constant "KK1" which is less than a second spring constant "KK2" of second biasing member 439b, the importance of which is detailed below.

The use of handle assembly 100 in conjunction with endoscopic assembly 400 is now detailed with reference to FIGS. 8-14 and 22-28. Initially, endoscopic assembly 400 is engaged with handle assembly 100, as detailed above. Since endoscopic assembly 400 is configured for ratcheting use of ratcheting drive assembly 130, ratchet pawl 142 remains disposed in the use position upon engagement of endoscopic assembly 400 with handle assembly 100. More specifically, due to the relative positions and dimensions of ring-shaped aperture 414 of proximal hub 410 and sleeve 152 of bypass assembly 150, as proximal hub 410 is inserted into receiver assembly 170, sleeve 152 is received within ring-shaped aperture 414, thereby enabling sleeve 152 to remain in its distal-most position under the bias of biasing member 154. With sleeve 152 remaining in its distal-most position, ratchet pawl 142 is retained in the use position under the bias of pawl biasing member 146. Thus, as detailed below, ratcheting use of handle assembly 100 and endoscopic assembly 400 is enabled. Once endoscopic assembly 400 and handle assembly 100 are engaged with ratchet pawl 142 remaining in the use position, handle assembly 100 and endoscopic assembly 400 are together ready for use.

In use, trigger 122 is initially disposed in the un-actuated position under the bias of biasing member 127. With trigger 122 disposed in the un-actuated position, drive bar 132 is disposed in a proximal-most position such that ratchet pawl 142 is disposed within distal recess 138 of drive bar 132. Further, with drive bar 132 disposed in the proximal-most position, inner shaft 431 of drive assembly 430 is disposed in a proximal-most position under the bias of first and second biasing members 439a, 439b, respectively. Thus, jaw members 460a, 460b, initially, are disposed in the open position. With jaw members 460a, 460b disposed in the open position, a new, unformed or open surgical clip (not shown) may be located or loaded within jaw members 460a, 460b, or may be otherwise operably positioned (manually or automatically) for insertion therebetween for formation or closure about tissue upon closure of jaw members 460a, 460b. For example, in some embodiments, during firing, a surgical clip is first advanced from elongated shaft 420 between jaw members 460a, 460b and, thereafter, jaw members 460a, 460b are closed to form the surgical clip. In such embodiments, a series of surgical clips may be loaded within elongated shaft 420 for sequential firing in a similar manner. However, other suitable surgical clips and/or configurations for firing thereof are also contemplated.

In order to close, fire, or form the surgical clip loaded between jaw members 460a, 460b, trigger 122 is urged from the un-actuated position to the actuated position. More specifically, grasping portion 123 of trigger 122 is pivoted towards fixed handle portion 112 of housing 110 to urge linkage 128 distally which, in turn, urges drive bar 132 distally. As drive bar 132 is urged distally, ratchet pawl 142 moves out of distal recess 138 of drive bar 132 and into engagement with ratchet rack 136. Once ratchet pawl 142 is engaged with ratchet rack 136, trigger 122 may not return towards the un-actuated position and, thus, drive bar 132 may not return proximally until trigger 122 reaches the actuated position, completing a full actuation stroke thereof.

As drive bar 132 is translated distally, drive bar 132 is advanced through housing 110, receiver assembly 170, and into bore 413 of proximal hub 410 of endoscopic assembly 400. Eventually, drive bar 132 contacts plunger 435 of drive assembly 430 of endoscopic assembly 400. Due to first spring constant "KK1" of first biasing member 439a being less than second spring constant "KK2" of second biasing member 439b, as drive bar 132 is initially urged into plunger 435, plunger 435 and inner shaft 431 translate together distally such that first biasing member 439a is compressed while second biasing member 439b remains substantially un-compressed. As inner shaft 431 is translated distally, a surgical clip is first loaded between first and second jaw members 460a, 460b and, thereafter, first and second jaw members 460a, 460b are transitioned from the open position to the closed position to form the surgical clip about tissue, although other configurations are also contemplated.

As noted above with respect to endoscopic assembly 300 (FIGS. 15-21), depending upon the particular endoscopic assembly used, the configuration of the surgical clip being formed, and/or other factors, the required travel distance of inner shaft 431 to fully form the surgical clip may vary. As also mentioned above, once ratchet pawl 142 is engaged with ratchet rack 136, trigger 122 may not return towards the un-actuated position until trigger 122 reaches the actuated position, completing a full actuation stroke thereof. Thus, in order to enable return of trigger 122 to the un-actuated position in instances where the required length of travel of drive bar 132 to fully form the surgical clip is insufficient for ratchet pawl 142 to clear ratchet rack 136 and enter proximal recess 139 of drive bar 132, endoscopic assembly 400 must allow further travel of drive bar 132, as detailed below.

As trigger 122 is further actuated to complete the full actuation stroke thereof, plunger 435 is continued to be driven distally. However, since inner shaft 431 cannot travel further distally, second biasing member 439b is compressed, thus allowing plunger 435 to translate distally independently of inner shaft 431. That is, the compression of second biasing member 439b enables inner shaft 431 to remain in position while the full actuation stroke of trigger 122 is completed.

Upon full actuation of trigger 122, e.g., upon reaching the actuated position of trigger 122, ratchet pawl 142 is moved into proximal recess 139 of drive bar 132. With ratchet pawl 142 disposed within proximal recess 139, trigger 122 may be released and returned to the un-actuated position under the bias of biasing member 127. Thereafter, the above-detailed use may be repeated to close, fire, or form additional surgical clips.

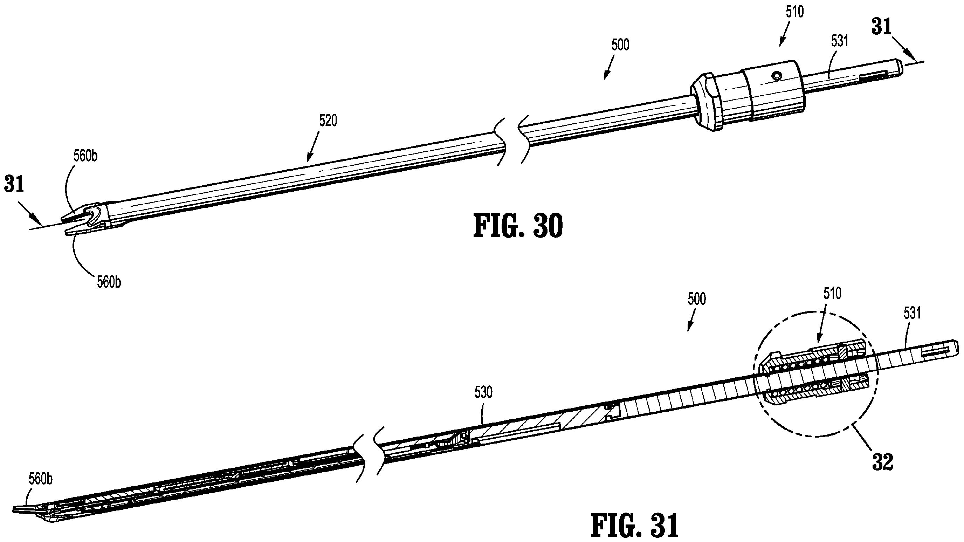

Referring to FIGS. 30-33, yet another embodiment of an endoscopic assembly is provided and generally identified by reference numeral 500. Endoscopic assembly 500 is configured for ratcheting use and, thus, upon engagement of endoscopic assembly 500 with handle assembly 100, as detailed above, ratchet pawl 142 remains in the use position to enable ratcheting use. Endoscopic assembly 500 generally includes a housing assembly 510, an elongated shaft 520 slidably supported within the housing assembly 510 and extending distally therefrom, a drive assembly 530 disposed within the elongated shaft 520, and a pair of jaw members 560a, 560b pivotably supported on a distal portion of the elongated shaft 520. Endoscopic assembly 500 is configured to close, fire, or form one or more surgical clips about tissue in a similar manner to endoscopic assembly 400, and therefore, in the interest of brevity, only the differences therebetween will be described in detail hereinbelow.

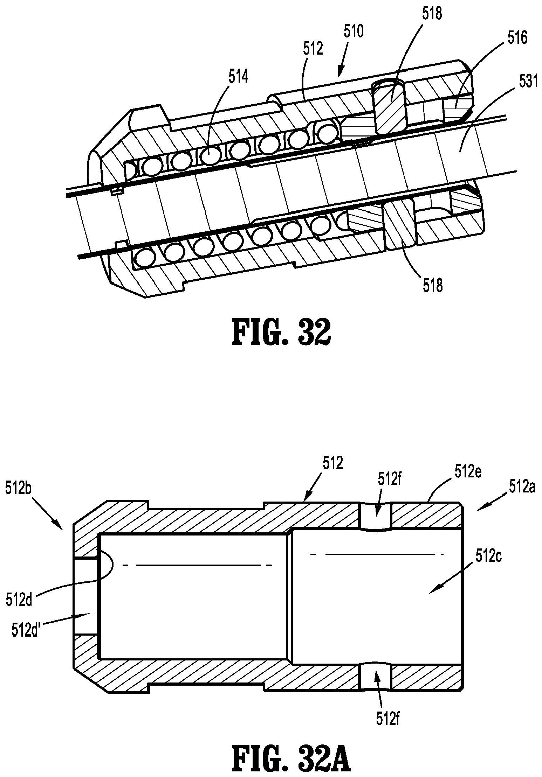

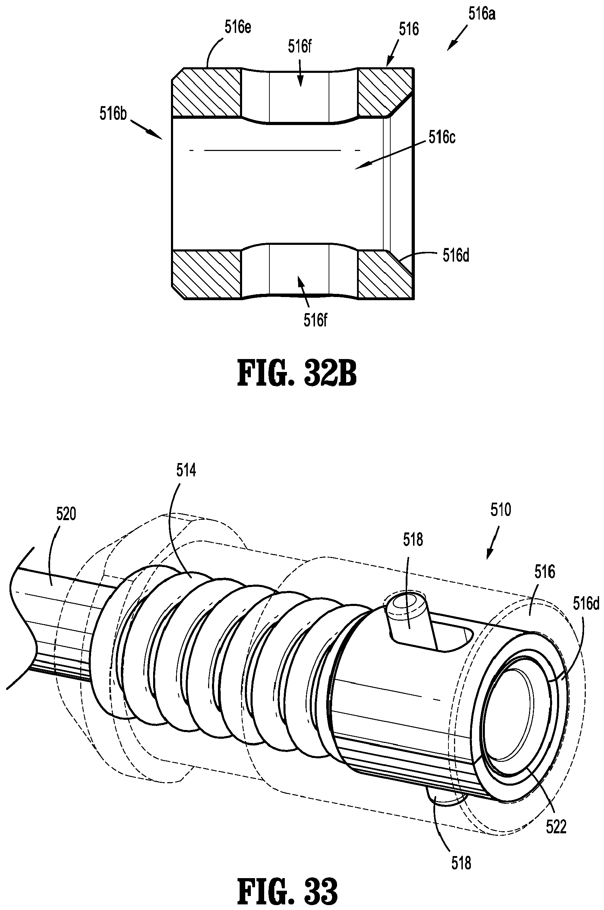

As best illustrated in FIG. 32, the housing assembly 510 generally includes a housing 512, biasing element 514, hub 516 and a pair of pins 518. The housing 512 defines a proximal portion 512a and a distal portion 512b and is configured to selectively engage the receiver assembly 170 of the handle assembly 100 in a similar manner to proximal hub 410 detailed hereinabove. Although generally illustrated as having a cylindrical configuration, it is contemplated that the housing 512 may include any suitable configuration capable of being received by the receiver assembly 170 of the handle assembly 100, such as square, rectangular, hexagonal, oval, or the like. The proximal portion 512a of the housing 512 defines a counterbore 512c (FIG. 32A) that extends in a distal direction and terminates in a proximal face 512d. The proximal face 512d defines a bore or opening 512d' extending through the distal portion 512b of the housing 512 that is configured to slidably receive the elongated shaft 520 therein, as will be described in further detail hereinbelow. An exterior surface 512e of the housing 512 defines a pair of opposed transverse through-holes 512f configured to fixedly receive the pair of pins 518 using any suitable means, such as interference fit, friction fit, adhesives, welding, or the like.

The hub 516 of the housing assembly 510 defines a proximal portion 516a and a distal portion 516b (FIG. 32B) and is configured to be slidably received within the counterbore 512c of the housing 512. In this manner, the hub 516 defines an external profile similar to a profile of the counterbore 512c of the housing 512, although it is contemplated that the hub 516 may define any suitable profile capable of being slidably received within the counterbore 512c. The proximal and distal portions 516a, 516b of the hub 516 define a through-hole 516c that extends therethrough and is configured to receive the elongated shaft 520 therein. The proximal portion 516a of the hub 516 defines a chamfer or bevel 516d configured to abut a corresponding flare 522 defined on a proximal portion of the elongated shaft 520, as will be described in further detail hereinbelow. An outer surface 516e of the hub 516 defines a pair of transverse, longitudinally extending, channels or slots 516f at a medial portion thereof that extends longitudinally therealong. The transverse channels 516f of the hub 516 are configured to slidably receive the pair of pins 518 in order to prevent rotation of the hub 516 with respect to the housing housing 512, as will be described in further detail hereinbelow. The transverse channels 516f of the hub 516 are configured to permit the hub 516 to translate in a proximal or distal direction with the counterbore 512c of the housing 512. In this manner, the transverse channels 516f act as a travel limiter for the hub 516, such that the pair of pins 518 abuts a proximal or distal portion of the transverse channels 516f to inhibit further movement in a proximal or distal direction.

The biasing element 514 of the housing assembly 510 is interposed between the proximal face 512d of the housing 512 and the distal portion 516b of the hub 516 and is concentrically disposed about the elongated shaft 520 (*e.g.,* the elongate shaft 520 passes through the biasing element 514). Although generally illustrated as being a coil spring, it is contemplated that the biasing element 514 may be any suitable biasing element or elements capable of being concentrically disposed over the elongated shaft 520, such as one or more Belleville washers, one or more curved disc springs, one or more wave washers, hydraulic, pneumatic, or the like. The biasing element 514 biases the hub 516 in a proximal direction to ensure that the bevel 516d of the hub 516 remains in contact with the flare 522 of the elongated shaft 520 and provides a biasing force against distal movement of the hub 516 and the elongated shaft 520. In this manner, the biasing element 514 includes a biasing force (*e.g.,* predetermined spring constant) sufficient to enable the first and second jaw members 560a, 560b to fully close and form a surgical clip, as will be described in further detail hereinbelow. As can be appreciated, the biasing element 514 includes a predetermined spring constant that is greater than the force required to close, fire, or form a surgical clip, but is sufficiently small to allow the biasing element to compress before the pair of jaws 560a, 560b become damaged or deformed, as will be described in further detail hereinbelow.

In operation, the endoscopic assembly 500 is utilized in a similar manner to that of endoscopic assembly 400, and therefore, for purposes of brevity, only the differences therebetween will be described in detail hereinbelow.

In operation, as the trigger 122 of the trigger assembly 120 is initially squeezed and the drive bar 132 is urged in a distal direction, an inner shaft 531 of the elongated shaft 520 is translated distally such that a surgical clip (not shown) is loaded between the first and second jaw members 560a, 560b. As the trigger 122 is further squeezed, the first and second jaw members 560a, 560b are transitioned from an open position to a closed or approximated position to form the surgical clip about the tissue.

As can be appreciated, closure of the jaws over large, thick, or dense tissue, or over a separate surgical clip may prevent closure of the first and second jaw members 560a, 560b, and therefore, inhibit the trigger 122 from returning to its original, un-actuated position. In this instance, the first and second jaw members 560a, 560b are also prevented from returning to an un-actuated position, thereby inhibiting the closure of any further surgical clips. Continued actuation of the trigger 122 causes an increasing amount of force to be translated along the inner shaft 531 of the elongated shaft 520, and ultimately, to the first and second jaw members 560a, 560b that are rotatably disposed on the elongated shaft 520. As can be appreciated, the increasing force applied to the first and second jaw members 560a, 560b may cause the first and second jaw members 560a, 560b to become deformed or otherwise damaged.

Thus, as the trigger 122 is further actuated, the force applied to the first and second jaw members 560a, 560b by the inner shaft 531 is translated to the elongated shaft 520 and biases the elongated shaft 520 in a distal direction (e.g., applies a force to the elongated shaft 520 in distal direction). In this manner, the flare 522 of the elongated shaft abuts the bevel 516d of the hub 516 and acts to compress the biasing element 514. Initially, the biasing force provided by the biasing element 514 inhibits distal advancement of the hub 516, and therefore, inhibits distal advancement of the elongated shaft 520. Continued actuation of the trigger 122 increases the force applied to the first and second jaw members 560a, 560b, and therefore, increases the force applied to the hub 516 in a distal direction, which overcomes the predetermined spring constant and causes the biasing element 514 to compress, allowing the hub 516, along with the elongated shaft 520, to be urged in a distal direction, preventing damage to the first and second jaw members 560a, 560b. Once the hub 516 has been urged in a distal direction a predetermined distance, the trigger 122 is permitted to return to the un-actuated position, and the surgical clip applier may be used for form another surgical clip.

It is contemplated that inner shaft 531 may be split into a proximal portion and a distal portion in a similar manner as disclosed above with respect to inner shaft 322. The components and operation of this embodiment of inner shaft 531 are similar to that of inner shaft 322, and therefore, for purposes of brevity, a detailed description of the components and operation thereof will not be described in detail hereinbelow.

It is contemplated, and within the scope of the present disclosure, that other endoscopic assemblies, including a pair of jaws having a unique and diverse closure stroke length thereof, may be provided for use with handle assembly 100 for ratcheting use or non-ratcheting use, similarly as detailed above with respect to endoscopic assemblies 300, 400, and 500 (FIGS. 15-21, 22-28, and 30-33, respectively). Such a configuration accommodates various different endoscopic assemblies having different configurations and/or different closure stroke lengths while providing a constant actuation stroke length of trigger 122. Accordingly, various endoscopic assemblies, constructed in accordance with the principles of the present disclosure, may be provided which are also capable of firing or forming or closing surgical clips of various sizes, materials, and configurations, across multiple platforms for multiple different manufactures.

Surgical instruments such as the clip appliers described herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

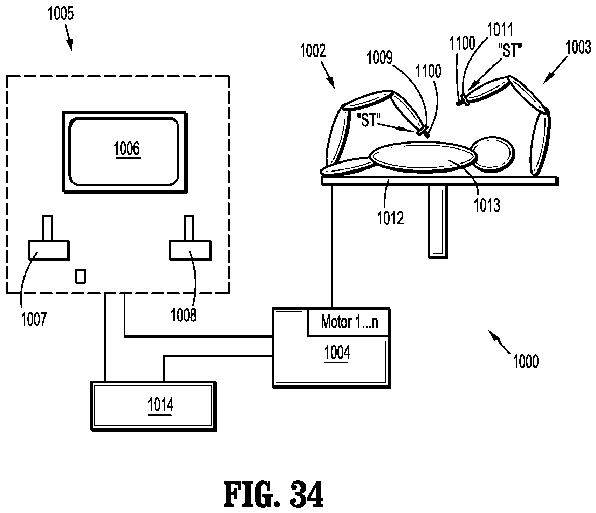

Referring to FIG. 34, a medical work station is shown generally as work station 1000 and generally may include a plurality of robot arms 1002, 1003; a control device 1004; and an operating console 1005 coupled with control device 1004. Operating console 1005 may include a display device 1006, which may be set up in particular to display three-dimensional images; and manual input devices 1007, 1008, by means of which a person (not shown), for example a surgeon, may be able to telemanipulate robot arms 1002, 1003 in a first operating mode.

Each of the robot arms 1002, 1003 may include a plurality of members, which are connected through joints, and an attaching device 1009, 1011, to which may be attached, for example, a surgical tool "ST" supporting an end effector 1100, in accordance with any one of several embodiments disclosed herein, as will be described in greater detail below.

Robot arms 1002, 1003 may be driven by electric drives (not shown) that are connected to control device 1004. Control device 1004 (e.g., a computer) may be set up to activate the drives, in particular by means of a computer program, in such a way that robot arms 1002, 1003, their attaching devices 1009, 1011 and thus the surgical tool (including end effector 1100) execute a desired movement according to a movement defined by means of manual input devices 1007, 1008. Control device 1004 may also be set up in such a way that it regulates the movement of robot arms 1002, 1003 and/or of the drives.

Medical work station 1000 may be configured for use on a patient 1013 lying on a patient table 1012 to be treated in a minimally invasive manner by means of end effector 1100. Medical work station 1000 may also include more than two robot arms 1002, 1003, the additional robot arms likewise being connected to control device 1004 and being telemanipulatable by means of operating console 1005. A medical instrument or surgical tool (including an end effector 1100) may also be attached to the additional robot arm. Medical work station 1000 may include a database 1014, in particular coupled to with control device 1004, in which are stored, for example, pre-operative data from patient/living being 1013 and/or anatomical atlases.

Reference is made herein to U.S. Patent No.8,828,023 to Neff et al., entitled "Medical Workstation," the entire content of which is incorporated herein by reference, for a more detailed discussion of the construction and operation of an exemplary robotic surgical system.

It is contemplated, and within the scope of the present disclosure, that other endoscopic assemblies, including a pair of jaws having a unique and diverse closure stroke length thereof, may be provided with a drive assembly, similar to any of the drive assemblies described herein, for accommodating and adapting the closure stroke length for the pair of jaws thereof to the constant trigger stroke length.

Accordingly, various endoscopic assemblies, constructed in accordance with the principles of the present disclosure, may be provided which are also capable of firing or forming or closing surgical clips of various sizes, materials, and configurations, across multiple platforms for multiple different manufactures.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods and techniques that are insubstantially different from those described above and/or in the appended claims are also intended to be within the scope of the disclosure.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods and techniques that are insubstantially different from those described above and/or in the appended claims are also intended to be within the scope of the disclosure.

The invention may be described by reference to the following numbered paragraphs:-
1. A reposable surgical clip applier, comprising:
   a shaft assembly including:
      an elongated shaft; and
      a housing assembly disposed about a proximal portion of the elongated shaft, the housing assembly including:
         a housing defining a counterbore; and
         a hub slidably disposed within the counterbore, the hub defining a throughhole configured to receive the elongated shaft therein, wherein the hub is operably coupled to the elongated shaft,
   wherein a force applied to the elongated shaft in a distal direction causes the hub to distally advance within the housing.
2. The reposable surgical clip applier according to paragraph 1, wherein the housing assembly further includes a biasing element interposed between the housing and the hub, wherein the biasing element includes a predetermined spring constant.
3. The reposable surgical clip applier according to paragraph 2, wherein when a force applied to the elongated shaft exceeds the predetermined spring constant the hub is distally advanced within the housing and compresses the biasing element.
4. The reposable surgical clip applier according to paragraph 3, wherein the shaft assembly further includes first and second jaw members pivotably disposed on a distal portion of the elongated shaft, the first and second jaw members configured to transition between a first, open position, to a second, approximated position.
5. The reposable surgical clip applier according to paragraph 4, wherein when the first and second jaw members are inhibited from transitioning to the second, approximated position, the elongated shaft and hub exert a force on the biasing element that exceeds the predetermined spring constant to distally advance the hub within the housing.
6. The reposable surgical clip applier according to paragraph 5, further including a handle assembly including a housing and a trigger rotatably supported on the handle assembly, the trigger being in mechanical communication with the first and second jaw members.
7. The reposable surgical clip applier according to paragraph 6, wherein the hub is configured to distally advance a predetermined distance to permit the trigger to return to a first, unactuated position, and therefore, permit the first and second jaw members to return to the first, open, position.
8. The reposable surgical clip applier according to paragraph 4, wherein the biasing element includes a predetermined spring constant that is greater than a force required to form a surgical clip disposed within the first and second jaw members.
9. The reposable surgical clip applier according to paragraph 1, wherein the housing defines a pair of transverse through-holes configured to fixedly receive a pair of pins.
10. The reposable surgical clip applier according to paragraph 9, wherein the hub defines a pair of transverse channels configured to slidably receive the pair of pins.
11. The reposable surgical clip applier according to paragraph 1, wherein a proximal portion of the elongated shaft defines a flare configured to abut a chamfer defined on a proximal portion of the hub, the flare configured to inhibit distal translation of the elongated shaft relative to the hub.
12. A shaft assembly for use with a reposable surgical clip applier, comprising:
   an elongated shaft; and
   a housing assembly disposed on a proximal portion of the elongated shaft, the housing assembly including:
      a housing defining a counterbore; and
      a hub slidably disposed within the counterbore, the hub defining a throughhole configured to receive the elongated shaft therein, wherein the hub is operably coupled to the elongated shaft,
   wherein a force applied to the elongated shaft in a distal direction causes the hub to distally advance within the housing.
13. The shaft assembly according to paragraph 12, wherein the housing assembly further includes a biasing element interposed between the housing and the hub, wherein the biasing element includes a predetermined spring constant.
14. The shaft assembly according to paragraph 13, wherein when a force applied to the elongated shaft exceeds the predetermined spring constant the hub is distally advanced within the housing and compresses the biasing element.
15. The shaft assembly according to paragraph 14, wherein the shaft assembly further includes first and second jaw members pivotably disposed on a distal portion of the elongated shaft, the first and second jaw members configured to transition between a first, open position, to a second, approximated position.
16. The shaft assembly according to paragraph 15, wherein when the first and second jaw members are inhibited from transitioning to the second, approximated position, the elongated shaft and hub exert a force on the biasing element that exceeds the predetermined spring constant to distally advance the hub within the housing.
17. The shaft assembly according to paragraph 15, wherein the biasing element includes a predetermined spring constant that is greater than a force required to form a surgical clip disposed within the first and second jaw members.
18. The shaft assembly according to paragraph 12, wherein the housing defines a pair of transverse through-holes configured to fixedly receive a pair of pins.
19. The shaft assembly according to paragraph 18, wherein the hub defines a pair of transverse channels configured to slidably receive the pair of pins.
20. The shaft assembly according to paragraph 12, wherein a proximal portion of the elongated shaft defines a flare configured to abut a chamfer defined on a proximal portion of the hub, the flare configured to inhibit distal translation of the elongated shaft relative to the hub.

## Claims

1. A reposable surgical clip applier, comprising:
a shaft assembly including:
an elongated shaft; and
a housing assembly disposed about a proximal portion of the elongated shaft, the housing assembly including:
a housing defining a counterbore; and
a hub slidably disposed within the counterbore, the hub defining a throughhole configured to receive the elongated shaft therein, wherein the hub is operably coupled to the elongated shaft,
wherein a force applied to the elongated shaft in a distal direction causes the hub to distally advance within the housing.

2. The reposable surgical clip applier according to claim 1, wherein the housing assembly further includes a biasing element interposed between the housing and the hub, wherein the biasing element includes a predetermined spring constant.

3. The reposable surgical clip applier according to claim 2, wherein when a force applied to the elongated shaft exceeds the predetermined spring constant the hub is distally advanced within the housing and compresses the biasing element; preferably wherein the shaft assembly further includes first and second jaw members pivotably disposed on a distal portion of the elongated shaft, the first and second jaw members configured to transition between a first, open position, to a second, approximated position.

4. The reposable surgical clip applier according to claim 3, wherein when the first and second jaw members are inhibited from transitioning to the second, approximated position, the elongated shaft and hub exert a force on the biasing element that exceeds the predetermined spring constant to distally advance the hub within the housing; preferably further including a handle assembly including a housing and a trigger rotatably supported on the handle assembly, the trigger being in mechanical communication with the first and second jaw members.

5. The reposable surgical clip applier according to claim 4, wherein the hub is configured to distally advance a predetermined distance to permit the trigger to return to a first, unactuated position, and therefore, permit the first and second jaw members to return to the first, open, position; and/or wherein the biasing element includes a predetermined spring constant that is greater than a force required to form a surgical clip disposed within the first and second jaw members.

6. The reposable surgical clip applier according to any preceding claim, wherein the housing defines a pair of transverse through-holes configured to fixedly receive a pair of pins; preferably wherein the hub defines a pair of transverse channels configured to slidably receive the pair of pins.

7. The reposable surgical clip applier according to any preceding claim, wherein a proximal portion of the elongated shaft defines a flare configured to abut a chamfer defined on a proximal portion of the hub, the flare configured to inhibit distal translation of the elongated shaft relative to the hub.

8. A shaft assembly for use with a reposable surgical clip applier, comprising:
an elongated shaft; and
a housing assembly disposed on a proximal portion of the elongated shaft, the housing assembly including:
a housing defining a counterbore; and
a hub slidably disposed within the counterbore, the hub defining a throughhole configured to receive the elongated shaft therein, wherein the hub is operably coupled to the elongated shaft,
wherein a force applied to the elongated shaft in a distal direction causes the hub to distally advance within the housing.

9. The shaft assembly according to claim 8, wherein the housing assembly further includes a biasing element interposed between the housing and the hub, wherein the biasing element includes a predetermined spring constant.

10. The shaft assembly according to claim 8 or claim 9, wherein when a force applied to the elongated shaft exceeds the predetermined spring constant the hub is distally advanced within the housing and compresses the biasing element; preferably wherein the shaft assembly further includes first and second jaw members pivotably disposed on a distal portion of the elongated shaft, the first and second jaw members configured to transition between a first, open position, to a second, approximated position.

11. The shaft assembly according to claim 10, wherein when the first and second jaw members are inhibited from transitioning to the second, approximated position, the elongated shaft and hub exert a force on the biasing element that exceeds the predetermined spring constant to distally advance the hub within the housing.

12. The shaft assembly according to claim 10 or claim 11, wherein the biasing element includes a predetermined spring constant that is greater than a force required to form a surgical clip disposed within the first and second jaw members.

13. The shaft assembly according to any of claims 8 to 12, wherein the housing defines a pair of transverse through-holes configured to fixedly receive a pair of pins.

14. The shaft assembly according to claim 13, wherein the hub defines a pair of transverse channels configured to slidably receive the pair of pins.

15. The shaft assembly according to any of claims 8 to 14, wherein a proximal portion of the elongated shaft defines a flare configured to abut a chamfer defined on a proximal portion of the hub, the flare configured to inhibit distal translation of the elongated shaft relative to the hub.
